# EUROPEAN PATENT APPLICATION

(11) **EP 4 269 570 A1**
(43) Date of publication of application: **01.11.2023**
(21) Application number: 21911008.7
(22) Date of filing: 23.12.2021
(51) Int. Cl.: C12N 5/10, C12N 15/864, C12N 15/867, C12Q 1/06

(54) **VIRAL VECTOR-PRODUCING CELLS HAVING IMPROVED ABILITY TO PRODUCE VECTOR, METHOD FOR PRODUCING SAME AND METHOD FOR SELECTING SAME**

(30) Priority: 25.12.2020 JP 2020217338
(71) Applicant: AGC INC., Chiyoda-ku, Tokyo 1008405 (JP)
(72) Inventor: MORIYAMA, Chinatsu, Tokyo 100-8405 (JP); KAWANO, Yasuhiro, Tokyo 100-8405 (JP); TANABE, Kana, Tokyo 100-8405 (JP); KUBOTA, Susumu, Tokyo 100-8405 (JP); KISHIMOTO, Megumi, Tokyo 100-8405 (JP)
(74) Representative: TBK
(86) International application number: PCT/JP2021/048034
(87) International publication number: WO 2022/138869

(57) **Abstract**

The present invention aims to provide viral vector-producing cells with improved ability to produce viral vector, a production method thereof, a kit containing the same, a method for selecting viral vector-producing cells, and the like. A viral vector-producing cell in which the expression of a protein derived from the viral vector-producing cell is regulated, specifically, the expression of the aforementioned protein is regulated as compared with that in the viral vector-producing cells before regulation of the expression or HEK293 cells (Accession number ATCC CRL1573) shows an improved ability to produce viral vectors.

## Description

### [Technical Field]

The present invention relates to viral vector-producing cells and a production method thereof, particularly, viral vector-producing cells with improved ability to produce vector, a production method thereof, a selection method thereof, a kit containing same, and the like.

### [Background Art]

In the medical field of gene therapy and the like, a method using a virus-derived vector for gene transfer (hereinafter viral vector) is generally used as a method for introducing genes into cells of mammals including human. A viral vector refers to a vector in which a naturally occurring virus is modified using genetic recombination technology to permit the transfer of a desired gene or the like into the target, and technological development is progressing in recent years.

As viruses from which viral vectors are derived, viruses with envelopes such as retroviruses, lentiviruses, Sendai viruses and herpes viruses, and viruses without envelopes (hereinafter non-enveloped virus) such as adenoviruses and adeno-associated viruses (hereinafter AAV) are well known.

In particular, AAV is considered promising as a gene transfer vector for use in gene therapy because it is capable of infecting cell types of a wide range of species, including human, can also infect non-dividing cells that have completed differentiation, has low risk of side effects due to lack of pathogenicity to humans, and has physicochemically stable viral particles.

Non-enveloped viral vectors such as adenoviruses and AAV have desirable characteristics for gene delivery, since they exhibit broad tissue affinity and cell affinity, capacity to accommodate large expression cassettes, and high transduction efficiency. Production of viral vector requires engineered cell lines capable of complementing the functions removed from the viral genome. For the development and commercial production of viral vector medicines, it is important to use cells that can be scaled up and can produce viral vectors with sufficient quality (safety) and purity.

For example, HEK293 cells are a cell line established by transforming human embryonic kidney cells with the adenovirus E1 gene, and are used for various purposes such as host for production of recombinant proteins and production and amplification of recombinant adenoviruses. However, its productivity remains problematic. On the other hand, HEK293T cells expressing SV40 Large T antigen in HEK293 cells improved the ability to produce viral vectors and increased productivity. However, there are concerns about clinical use thereof due to the risk of carcinogenicity/immunogenicity (Non Patent Literature 1).

Regarding the production of AAV vectors, efforts have been made to obtain high-titer AAV vector solutions by using cells with miRNA artificially introduced thereinto (Patent Literature 1). However, the amount of AAV vectors produced is still insufficient, and the development of a production method that achieves higher productivity is demanded.

### [Citation List]

### [Patent Literature]

[PTL 1]
JP-B-6093358

### [Non Patent Literature]

[NPL 1]
Dahae Hailey Bae et al., (2020) Mol Ther Methods Clin Dev. 2020 Sep 11; 18: 631-638

### [Summary of Invention]

### [Technical Problem]

The present invention aims to provide viral vector-producing cells with improved ability to produce viral vector, a production method thereof, a selection method thereof, a kit containing same, and the like.

### [Solution to Problem]

In view of the above-mentioned problems, the present inventors have compared the protein expression profiles of viral vector-producing cells showing high viral vector productivity (hereinafter to be also referred to as viral vector high-producing cells or simply, high-producing cells) and viral vector-producing cells showing low productivity (hereinafter to be also referred to as viral vector low-producing cells or simply, low-producing cells) to search for proteins with deviation of expression levels between samples. As a result, they have found some proteins showing increased expression levels and some proteins showing decreased expression levels, in viral vector high-producing cells. Using these proteins as candidate proteins to be targeted for expression regulation in viral vector-producing cells, they have expressed or promoted the proteins, or inhibited or suppressed the proteins, and successfully obtained viral vector-producing cells with high viral vector-producing ability, which resulted in the completion of the present invention.

Accordingly, the present invention provides the following.

[1] A viral vector-producing cell with an improved ability to produce vectors, wherein expression of a protein derived from the viral vector-producing cell in the cell is regulated.
[2] The viral vector-producing cell of the above-mentioned [1], wherein the expression of the protein derived from the viral vector-producing cell is regulated as compared with that in the viral vector-producing cell before regulation of the expression of the aforementioned protein.
[3] The viral vector-producing cell of the above-mentioned [1], wherein the expression of the protein derived from the viral vector-producing cell is regulated as compared with that in HEK293 cell (Accession number ATCC CRL1573).
[4] The viral vector-producing cell of the above-mentioned [1] or [2], wherein the aforementioned expression of the protein derived from the viral vector-producing cell is increased as compared with that in the viral vector-producing cell before regulation of the expression of the aforementioned protein.
[5] The viral vector-producing cell of the above-mentioned [4], wherein the aforementioned expression of the protein derived from the viral vector-producing cell is increased 1.5 times or more at the time of viral vector production, as compared with that in the viral vector-producing cell before regulation of the expression of the aforementioned protein.
[6] The viral vector-producing cell of the above-mentioned [1] or [3], wherein the aforementioned expression of the protein derived from the viral vector-producing cell is increased as compared with that in HEK293 cell (Accession number ATCC CRL1573).
[7] The viral vector-producing cell of the above-mentioned [6], wherein the aforementioned expression of the protein derived from the viral vector-producing cell is increased 1.5 times or more at the time of viral vector production, as compared with that in the HEK293 cell (Accession number ATCC CRL1573).
[8] The viral vector-producing cell of any of the above-mentioned [4] to [7], wherein the increase in the aforementioned expression of the protein derived from the viral vector-producing cell is achieved by addition of an additive to the cell.
[9] The viral vector-producing cell of any of the above-mentioned [4] to [7], wherein the increase in the aforementioned expression of the protein derived from the viral vector-producing cell is achieved by introducing an exogenous plasmid into the viral vector-producing cell.
[10] The viral vector-producing cell of any of the above-mentioned [4] to [9], wherein the aforementioned protein derived from viral vector-producing cell is at least one type selected from the group consisting of PIK3C2A, UBE2C, ASNS, NIFK, PDCD4, DDX47, GTPBP4, FTSJ3, DDX49, GNL2, NAT10, UBL5, SERBP1, and NOVA2.
[11] The viral vector-producing cell of any of the above-mentioned [4] to [10], wherein the aforementioned protein derived from viral vector-producing cell is at least one type selected from the group consisting of PIK3C2A, UBE2C, ASNS, NIFK, GTPBP4, FTSJ3, DDX49, GNL2, SERBP1, and NOVA2.
[12] The viral vector-producing cell of any of the above-mentioned [4] to [11], wherein the aforementioned proteins derived from viral vector-producing cell are NIFK and NOVA2, or SERBP1 and NOVA2.
[13] The viral vector-producing cell of the above-mentioned [1] or [2], wherein the aforementioned expression of the protein derived from the viral vector-producing cell is decreased as compared with that in the viral vector-producing cell before regulation of the expression of the aforementioned protein.
[14] The viral vector-producing cell of the above-mentioned [13], wherein the aforementioned expression of the protein derived from the viral vector-producing cell is decreased by not less than 10% at the time of viral vector production, as compared with that in the viral vector-producing cell before regulation of the expression of the aforementioned protein.
[15] The viral vector-producing cell of the above-mentioned [1] or [3], wherein the aforementioned expression of the protein derived from the viral vector-producing cell is decreased as compared with that in HEK293 cell (Accession number ATCC CRL1573).
[16] The viral vector-producing cell of the above-mentioned [15], wherein the aforementioned expression of the protein derived from the viral vector-producing cell is decreased by not less than 10% at the time of viral vector production, as compared with that in the HEK293 cell (Accession number ATCC CRL1573).
[17] The viral vector-producing cell of any of the above-mentioned [13] to [16], wherein the decrease in the aforementioned expression of the protein derived from the viral vector-producing cell is achieved by addition of an additive to the cell.
[18] The viral vector-producing cell of any of the above-mentioned [13] to [16], wherein the decrease in the aforementioned expression of the protein derived from the viral vector-producing cell is achieved by introducing an exogenous plasmid into the viral vector-producing cell.
[19] The viral vector-producing cell of any of the above-mentioned [13] to [18], wherein the aforementioned protein derived from viral vector-producing cell is at least one type selected from the group consisting of ISG15, CD44, LMNA, ACP2 and MRE11.
[20] The viral vector-producing cell of any of the above-mentioned [13] to [19], wherein the aforementioned protein derived from viral vector-producing cell is at least one type selected from the group consisting of ISG15, CD44, and MRE11.
[21] The viral vector-producing cell of any of the above-mentioned [1] to [20], which is derived from an animal cell.
[22] The viral vector-producing cell of any of the above-mentioned [1] to [21], which is derived from a human cell.
[23] The viral vector-producing cell of the above-mentioned [22], wherein the aforementioned human cell is HEK293 cell.
[24] The viral vector-producing cell of any of the above-mentioned [1] to [23], wherein the aforementioned viral vector is derived from a virus belonging to Parvoviridae or Retroviridae.
[25] The viral vector-producing cell of any of the above-mentioned [1] to [23], wherein the aforementioned viral vector is an adeno-associated viral vector.
[26] A method for producing a viral vector-producing cell with an improved ability to produce vectors, comprising regulating expression of a protein derived from the viral vector-producing cell in the viral vector-producing cell.
[27] The production method of the above-mentioned [26], wherein the regulation is to regulate the expression of the aforementioned protein by comparison with the viral vector-producing cell before regulation.
[28] The production method of the above-mentioned [26], wherein the regulation is to regulate the expression of the aforementioned protein by comparison with HEK293 cell (Accession number ATCC CRL1573).
[29] The production method of the above-mentioned [26] or [27], wherein the regulation is to increase the aforementioned expression of the protein derived from the viral vector-producing cell as compared with that in the viral vector-producing cell before regulation of the expression of the aforementioned protein.
[30] The production method of the above-mentioned [29], wherein the regulation is to increase the aforementioned expression of the protein derived from the viral vector-producing cell 1.5 times or more at the time of viral vector production, as compared with that in the viral vector-producing cell before regulation of the expression of the aforementioned protein.
[31] The production method of the above-mentioned [26] or [28], wherein the regulation is to increase the aforementioned expression of the protein derived from the viral vector-producing cell, as compared with that in HEK293 cell (Accession number ATCC CRL1573).
[32] The production method of the above-mentioned [31], wherein the regulation is to increase the aforementioned expression of the protein derived from the viral vector-producing cell 1.5 times or more at the time of viral vector production, as compared with that in the HEK293 cell (Accession number ATCC CRL1573).
[33] The production method of any of the above-mentioned [29] to [32], wherein the increase in the aforementioned expression of the protein derived from the viral vector-producing cell is achieved by addition of an additive to the cell.
[34] The production method of any of the above-mentioned [29] to [32], wherein the increase in the aforementioned expression of the protein derived from the viral vector-producing cell is achieved by introducing an exogenous plasmid into the viral vector-producing cell.
[35] The production method of any of the above-mentioned [29] to [34], wherein the aforementioned protein derived from the viral vector-producing cell is at least one type selected from the group consisting of PIK3C2A, UBE2C, ASNS, NIFK, PDCD4, DDX47, GTPBP4, FTSJ3, DDX49, GNL2, NAT10, UBL5, SERBP1, and NOVA2.
[36] The production method of any of the above-mentioned [29] to [35], wherein the aforementioned protein derived from viral vector-producing cell is at least one type selected from the group consisting of PIK3C2A, UBE2C, ASNS, NIFK, GTPBP4, FTSJ3, DDX49, GNL2, SERBP1, and NOVA2.
[37] The production method of any of the above-mentioned [29] to [36], wherein the proteins derived from aforementioned viral vector-producing cell are NIFK and NOVA2, or SERBP1 and NOVA2.
[38] The production method of the above-mentioned [26] or [27], wherein the regulation is to decrease the aforementioned expression of the protein derived from the viral vector-producing cell as compared with that in the viral vector-producing cell before regulation of the expression of the aforementioned protein.
[39] The production method of the above-mentioned [38], wherein the regulation is to decrease the aforementioned expression of the protein derived from the viral vector-producing cell by not less than 10% at the time of viral vector production, as compared with that in the viral vector-producing cell before regulation of the expression of the aforementioned protein.
[40] The production method of the above-mentioned [26] or [28], wherein the aforementioned expression of the protein derived from the viral vector-producing cell is decreased as compared with that in the HEK293 cell (Accession number ATCC CRL1573).
[41] The production method of the above-mentioned [40], wherein the regulation is to decrease the aforementioned expression of the protein derived from the viral vector-producing cell by not less than 10% at the time of viral vector production, as compared with that in the HEK293 cell (Accession number ATCC CRL1573).
[42] The production of any of the above-mentioned [26] to [41], wherein the decrease in the aforementioned expression of the protein derived from the viral vector-producing cell is achieved by addition of an additive to the cell.
[43] The production method of any of the above-mentioned [26] to [41], wherein the decrease in the aforementioned expression of the protein derived from the viral vector-producing cell is achieved by introducing an exogenous plasmid into the viral vector-producing cell.
[44] The production method of any of the above-mentioned [26] to [43], wherein the aforementioned protein derived from viral vector-producing cell is at least one type selected from the group consisting of ISG15, CD44, LMNA, ACP2 and MRE11.
[45] The production method of any of the above-mentioned [26] to [44], wherein the aforementioned protein derived from viral vector-producing cell is at least one type selected from the group consisting of ISG15, CD44, and MRE11.
[46] The production method of any of the above-mentioned [26] to [45], wherein the cell is derived from an animal cell.
[47] The production method of any of the above-mentioned [26] to [46], wherein the cell is derived from a human cell.
[48] A method for producing the viral vector-producing cell of the above-mentioned [47], wherein the aforementioned human cell is HEK293 cell.
[49] The production method of any of the above-mentioned [26] to [48], wherein the aforementioned viral vector is derived from a virus belonging to Parvoviridae or Retroviridae.
[50] The production method of any of the above-mentioned [26] to [48], wherein the aforementioned viral vector is an adeno-associated viral vector.
[51] A method for enhancing a production amount of a viral vector in a viral vector-producing cell, comprising regulating expression of a protein derived from the viral vector-producing cell in the viral vector-producing cell.
[52] The method of the above-mentioned [51], wherein the regulation is performed by comparison with the viral vector-producing cell before regulation of the expression of the aforementioned protein.
[53] The method of the above-mentioned [51], wherein the regulation is performed by comparison with HEK293 cell (Accession number ATCC CRL1573).
[54] The method of the above-mentioned [51] or [52], wherein the regulation is to increase the aforementioned expression of the protein derived from the viral vector-producing cell as compared with that in the viral vector-producing cell before regulation of the expression of the aforementioned protein.
[55] The method of the above-mentioned [54], wherein the regulation is to increase the aforementioned expression of the protein derived from the viral vector-producing cell 1.5 times or more at the time of viral vector production, as compared with that in the viral vector-producing cell before regulation of the expression of the aforementioned protein.
[56] The method of the above-mentioned [51] or [53], wherein the regulation is to increase the aforementioned expression of the protein derived from the viral vector-producing cell, as compared with that in HEK293 cell (Accession number ATCC CRL1573).
[57] The method of the above-mentioned [56], wherein the regulation is to increase the aforementioned expression of the protein derived from the viral vector-producing cell 1.5 times or more at the time of viral vector production, as compared with that in the HEK293 cell (Accession number ATCC CRL1573).
[58] The method of any of the above-mentioned [54] to [57], wherein the increase in the aforementioned expression of the protein derived from the viral vector-producing cell is achieved by addition of an additive to the cell.
[59] The method of any of the above-mentioned [54] to [57], wherein the increase in the aforementioned expression of the protein derived from the viral vector-producing cell is achieved by introducing an exogenous plasmid into the viral vector-producing cell.
[60] The method of any of the above-mentioned [54] to [59], wherein the protein derived from aforementioned viral vector-producing cell is at least one type selected from the group consisting of PIK3C2A, UBE2C, ASNS, NIFK, PDCD4, DDX47, GTPBP4, FTSJ3, DDX49, GNL2, NAT10, UBL5, SERBP1, and NOVA2.
[61] The method of any of the above-mentioned [54] to [60], wherein the protein derived from aforementioned viral vector-producing cell is at least one type selected from the group consisting of PIK3C2A, UBE2C, ASNS, NIFK, GTPBP4, FTSJ3, DDX49, GNL2, SERBP1, and NOVA2.
[62] The method of any of the above-mentioned [54] to [61], wherein the proteins derived from aforementioned viral vector-producing cell are NIFK and NOVA2, or SERBP1 and NOVA2.
[63] The method of the above-mentioned [51] or [52], wherein the regulation is to decrease the aforementioned expression of the protein derived from the viral vector-producing cell as compared with that in the viral vector-producing cell before regulation of the expression of the aforementioned protein.
[64] The method of the above-mentioned [63], wherein the regulation is to decrease the aforementioned expression of the protein derived from the viral vector-producing cell by not less than 10% at the time of viral vector production, as compared with that in the viral vector-producing cell before regulation of the expression of the aforementioned protein.
[65] The method of the above-mentioned [51] or [53], wherein the aforementioned expression of the protein derived from the viral vector-producing cell is decreased as compared with that in the HEK293 cell (Accession number ATCC CRL1573).
[66] The method of the above-mentioned [65], wherein the regulation is to decrease the aforementioned expression of the protein derived from the viral vector-producing cell by not less than 10% at the time of viral vector production, as compared with that in the HEK293 cell (Accession number ATCC CRL1573).
[67] The method of any of the above-mentioned [63] to [66], wherein the decrease in the aforementioned expression of the protein derived from the viral vector-producing cell is achieved by addition of an additive to the cell.
[68] The method of any of the above-mentioned [63] to [66], wherein the decrease in the aforementioned expression of the protein derived from the viral vector-producing cell is achieved by introducing an exogenous plasmid into the viral vector-producing cell.
[69] The method of any of the above-mentioned [63] to [68], wherein the protein derived from aforementioned viral vector-producing cell is at least one type selected from the group consisting of ISG15, CD44, LMNA, ACP2 and MRE11.
[70] The method of any of the above-mentioned [63] to [69], wherein the protein derived from aforementioned viral vector-producing cell is at least one type selected from the group consisting of ISG15, CD44, and MRE11.
[71] The method of any of the above-mentioned [51] to [70], wherein the cell is derived from an animal cell.
[72] The method of any of the above-mentioned [51] to [71], wherein the cell is derived from a human cell.
[73] The method of the above-mentioned [72], wherein the aforementioned human cell is HEK293 cell.
[74] The method of any of the above-mentioned [51] to [73], wherein the aforementioned viral vector is derived from a virus belonging to Parvoviridae or Retroviridae.
[75] The method of any of the above-mentioned [51] to [74], wherein the aforementioned viral vector is an adeno-associated viral vector.
[76] A kit for producing a viral vector, comprising the viral vector-producing cells of any of the above-mentioned [1] to [25] or viral vector-producing cells produced by the production method of any of the above-mentioned [26] to [50].
[77] A method for selecting a viral vector-producing cell with an improved ability to produce vectors, comprising a step of selecting a cell in which the expression of a protein derived from the viral vector-producing cell is regulated in the viral vector-producing cell.
[78] The method of the above-mentioned [77], comprising a step of selecting a cell in which expression of the protein derived from the viral vector-producing cell is regulated in the viral vector-producing cells as compared with that in the viral vector-producing cell before regulation of the expression of the aforementioned protein.
[79] The method of the above-mentioned [77], comprising a step of selecting a cell in which expression of the protein derived from the viral vector-producing cell is regulated in the viral vector-producing cells as compared with that in HEK293 cell (Accession number ATCC CRL1573).
[80] The method of the above-mentioned [77] or [78], wherein the regulation is performed to increase the expression as compared with that in the viral vector-producing cell before regulation of the expression of the aforementioned protein.
[81] The method of the above-mentioned [77] or [79], wherein the regulation is performed to increase the expression as compared with that in the HEK293 cell (Accession number ATCC CRL1573).
[82] The method of the above-mentioned [77] or [78], wherein the regulation is performed to decrease the aforementioned expression of the protein derived from the viral vector-producing cell as compared with that in the viral vector-producing cell before regulation of the expression of the aforementioned protein.
[83] The method of the above-mentioned [77] or [79], wherein the regulation is performed to decrease the aforementioned expression of the protein derived from the viral vector-producing cell, as compared with that in HEK293 cell (Accession number ATCC CRL1573).
[84] The method of the above-mentioned [82] or [83], wherein the protein derived from the aforementioned viral vector-producing cells is CD44.

### [Advantageous Effects of Invention]

The viral vector-producing cells in the present invention show high viral vector producing ability. Therefore, it becomes possible to produce more viral vectors by using the viral vector-producing cell of the present invention as a packaging cell. Since the amount that can be produced at one time increases, the number of lots in production can be reduced, which makes it possible to reduce quality variations between lots.

### [Brief Description of Drawings]

[Fig. 1]

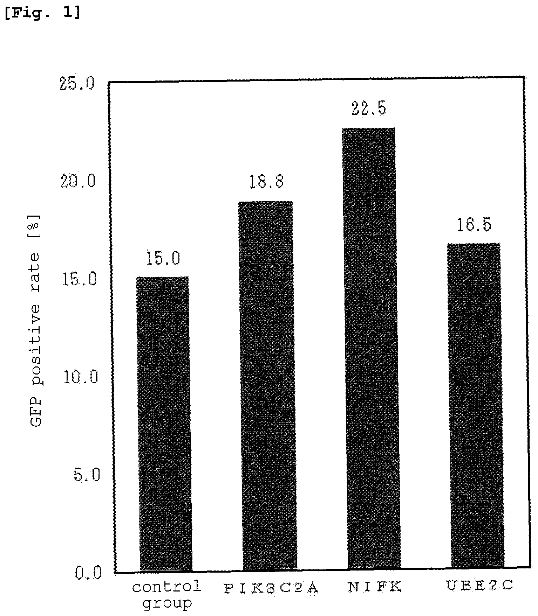

   Fig. 1 is a graph evaluating the gene transfer efficiency of cell fractions of AAV2 vectors produced by cells that transiently expressed PIK3C2A, NIFK, or UBE2C during AAV2 vector production (indicated by GFP positive rate).
[Fig. 2]

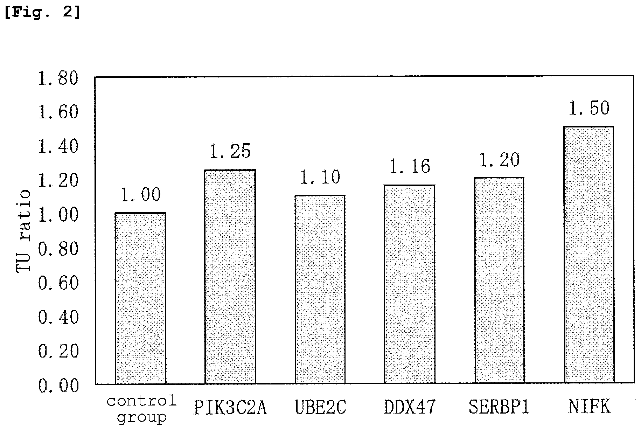

   Fig. 2 is a graph evaluating the gene transfer efficiency of cell fractions of AAV2 vectors produced by cells that transiently expressed PIK3C2A, UBE2C, DDX47, SERBP1 or NIFK during AAV2 vector production (indicated by ratio of GFP positive rate with respect to control group).
[Fig. 3]

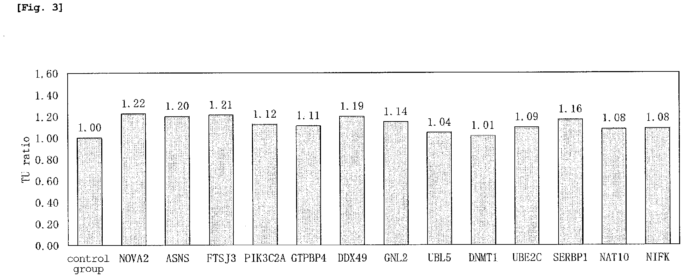

   Fig. 3 is a graph evaluating the gene transfer efficiency when AAV2 vectors, which were produced by cells that transiently expressed NOVA2, ASNS, FTSJ3, PIK3C2A, GTPBP4, DDX49, GNL2, UBL5, DNMT1, UBE2C, SERBP1, NAT10, or NIFK during AAV2 vector production, were extracted with surfactant (indicated by ratio of GFP positive rate with respect to control group).
[Fig. 4]

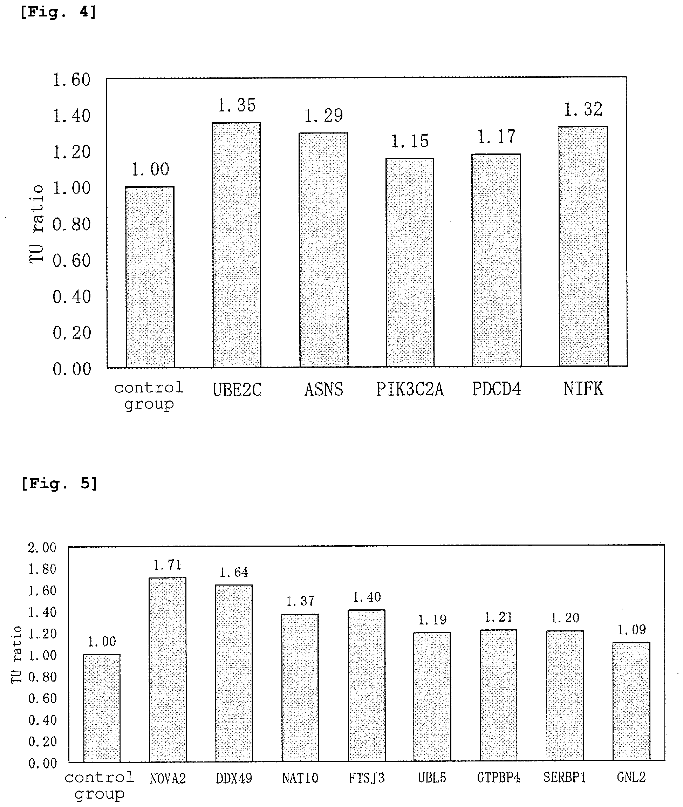

   Fig. 4 is a graph evaluating the gene transfer efficiency of supernatant fractions of AAV1 vectors produced by cells that transiently expressed UBE2C, ASNS, PIK3C2A, PDCD4, or NIFK during AAV1 vector production (indicated by ratio of GFP positive rate with respect to control group).
[Fig. 5]
   Fig. 5 is a graph evaluating the gene transfer efficiency when AAV1 vectors, which were produced by cells that transiently expressed NOVA2, DDX49, NAT10, FTSJ3, UBL5, GTPBP4, SERBP1, or GNL2 during AAV1 vector production, were extracted with surfactant (indicated by ratio of GFP positive rate with respect to control group).
[Fig. 6]

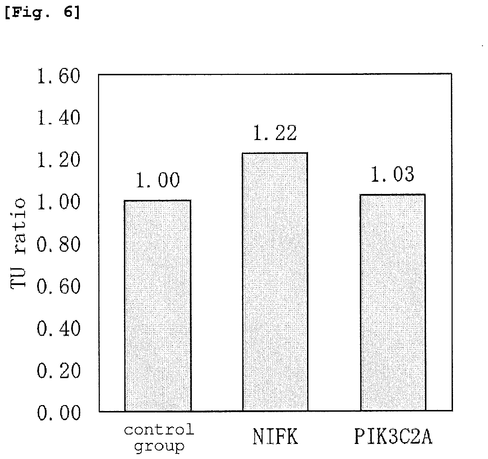

   Fig. 6 is a graph evaluating the gene transfer efficiency when AAV6 vectors, which were produced by cells that transiently expressed NIFK or PIK3C2A during AAV6 vector production, were extracted with surfactant (indicated by ratio of GFP positive rate with respect to control group).
[Fig. 7]

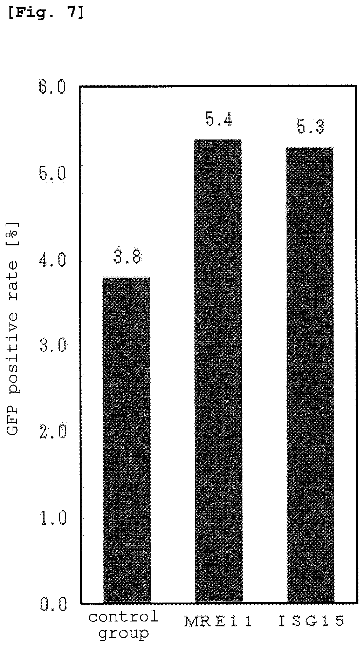

   Fig. 7 is a graph evaluating the gene transfer efficiency of cell fractions of AAV2 vectors produced by cells in which MRE11 or ISG15 was knocked down during AAV2 vector production (indicated by GFP positive rate).
[Fig. 8]

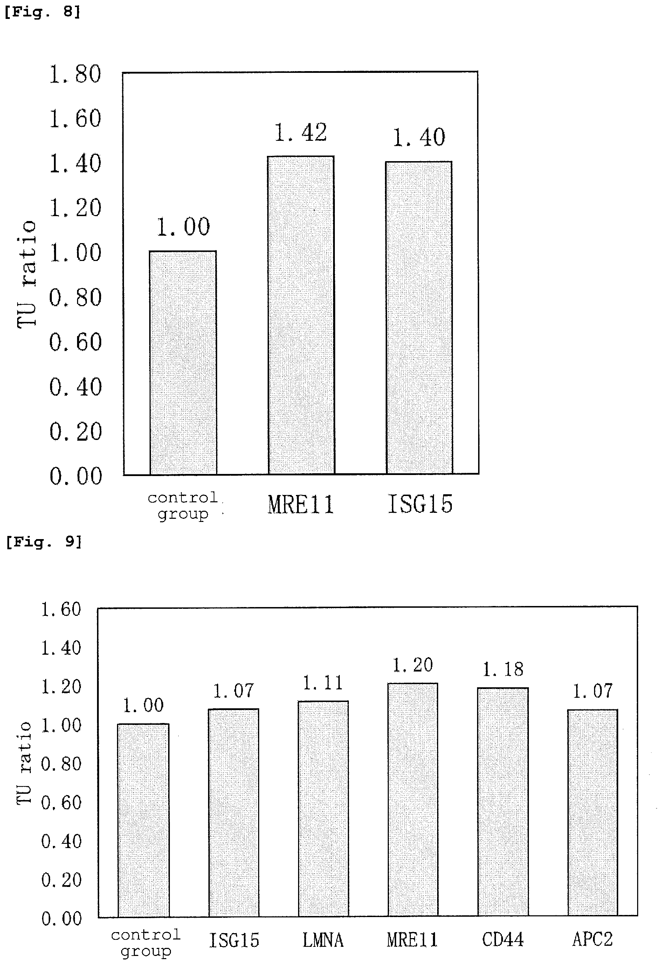

   Fig. 8 is a graph evaluating the gene transfer efficiency of cell fractions of AAV2 vectors produced by cells in which MRE11 or ISG15 was knocked down during AAV2 vector production (indicated by ratio of GFP positive rate with respect to control group).
[Fig. 9]
   Fig. 9 is a graph evaluating the gene transfer efficiency when AAV2 vectors, which were produced by cells in which ISG15, LMNA, MRE11, CD44, or APC2 was knocked down during AAV2 vector production, were extracted with surfactant (indicated by ratio of GFP positive rate with respect to control group).
[Fig. 10]

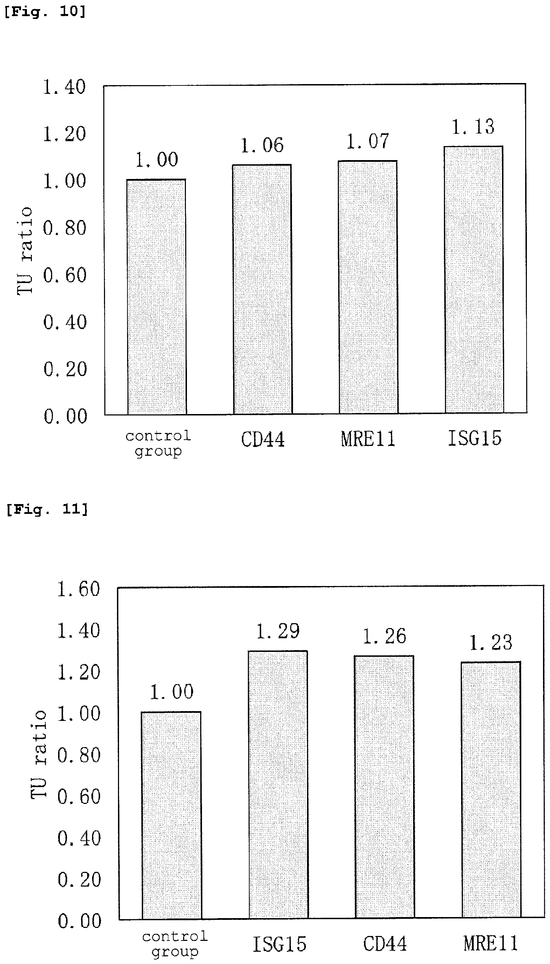

   Fig. 10 is a graph evaluating the gene transfer efficiency when AAV1 vectors, which were produced by cells in which CD44, MRE11, or ISG15 was knocked down during AAV1 vector production, was extracted with surfactant (indicated by ratio of GFP positive rate with respect to control group).
[Fig. 11]
   Fig. 11 is a graph evaluating the gene transfer efficiency when AAV6 vectors, which were produced by cells in which ISG15, CD44, or MRE11 was knocked down during AAV6 vector production, were extracted with surfactant (indicated by ratio of GFP positive rate with respect to control group).
[Fig. 12]

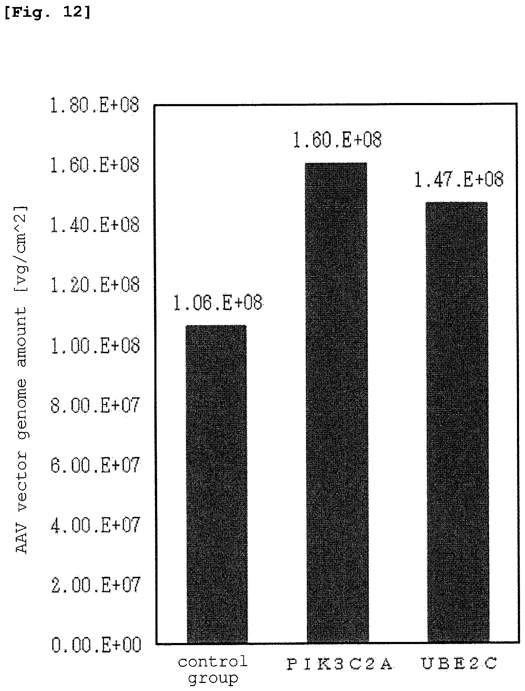

   Fig. 12 is a graph evaluating the total amount of AAV vector genomes when AAV2 vectors, which were produced by cells that transiently expressed PIK3C2A or UBE2C during AAV2 vector production, was sampled separately into a cell fraction and a supernatant fraction (indicated by AAV vector production amount per unit area).
[Fig. 13]

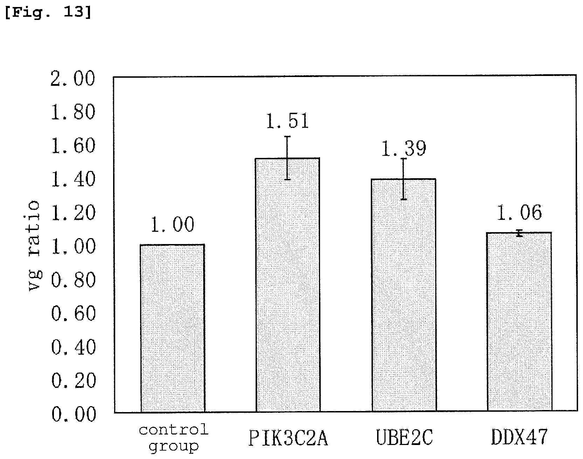

   Fig. 13 is a graph evaluating the total amount of AAV vector genomes when AAV2 vectors, which were produced by cells that transiently expressed PIK3C2A, UBE2C, or DDX47 during AAV2 vector production, were sampled separately into a cell fraction and a supernatant fraction (indicated by ratio of AAV vector production amount per unit area with respect to control group).
[Fig. 14]

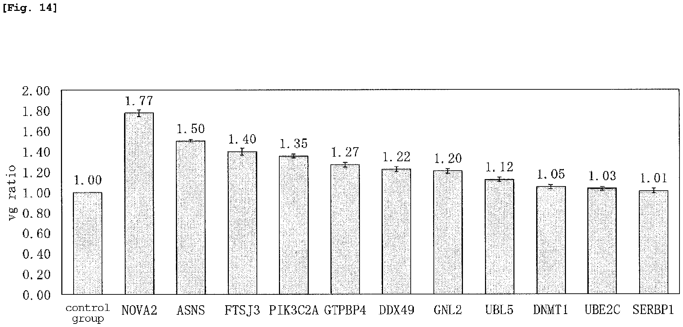

   Fig. 14 is a graph evaluating the amount of AAV vector genomes when AAV2 vectors, which were produced by cells that transiently expressed NOVA2, ASNS, FTSJ3, PIK3C2A, GTPBP4, DDX49, GNL2, UBL5, DNMT1, UBE2C, or SERBP1 during AAV2 vector production, were extracted with surfactant (indicated by ratio of AAV vector production amount per unit area with respect to control group).
[Fig. 15]

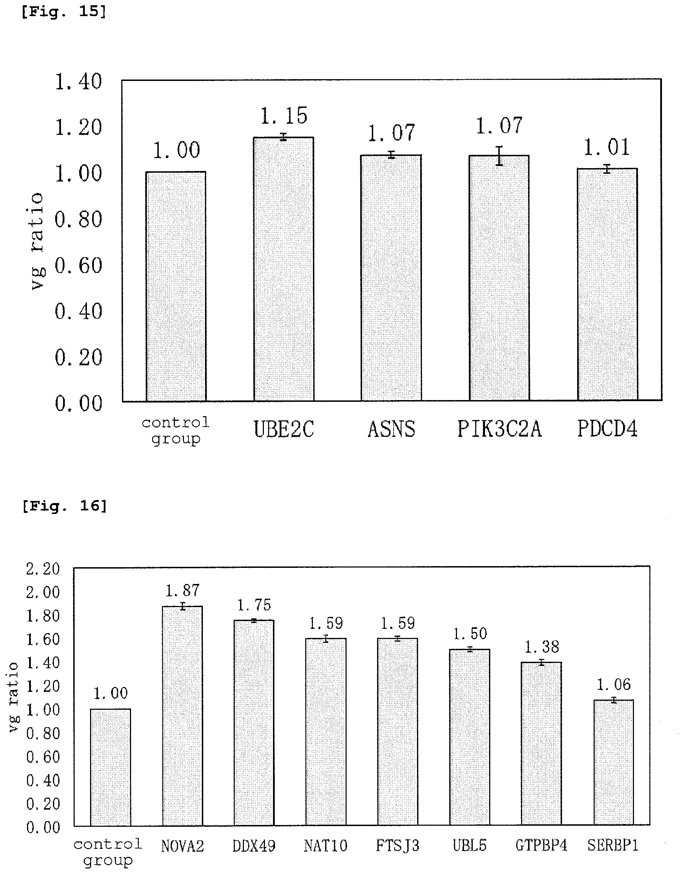

   Fig. 15 is a graph evaluating the total amount of AAV vector genomes when AAV1 vectors, which were produced by cells that transiently expressed UBE2C, ASNS, PIK3C2A, or PDCD4 during AAV1 vector production, were sampled separately into a cell fraction and a supernatant fraction (indicated by ratio of AAV vector production amount per unit area with respect to control group).
[Fig. 16] Fig. 16 is a graph evaluating the amount of AAV vector genomes when AAV1 vectors, which were produced by cells that transiently expressed NOVA2, DDX49, NAT10, FTSJ3, UBL5, GTPBP4, or SERBP1 during AAV1 vector production, were extracted with surfactant (indicated by ratio of AAV vector production amount per unit area with respect to control group).
[Fig. 17]

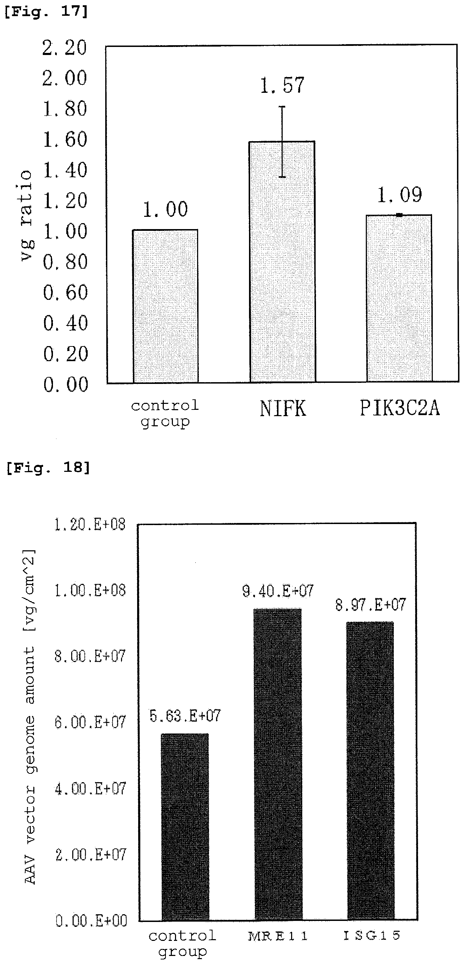

   Fig. 17 is a graph evaluating the amount of AAV genomes when AAV6 vectors, which were produced by cells that transiently expressed NIFK or PIK3C2A during AAV6 vector production, were extracted with surfactant (indicated by ratio of AAV vector production amount per unit area with respect to control group).
[Fig. 18]
   Fig. 18 is a graph evaluating the total amount of AAV vector genomes when AAV2 vectors, which were produced by cells in which MRE11 or ISG15 was knocked down during AAV2 vector production, were sampled separately into a cell fraction and a supernatant fraction (indicated by AAV vector production amount per unit area).
[Fig. 19]

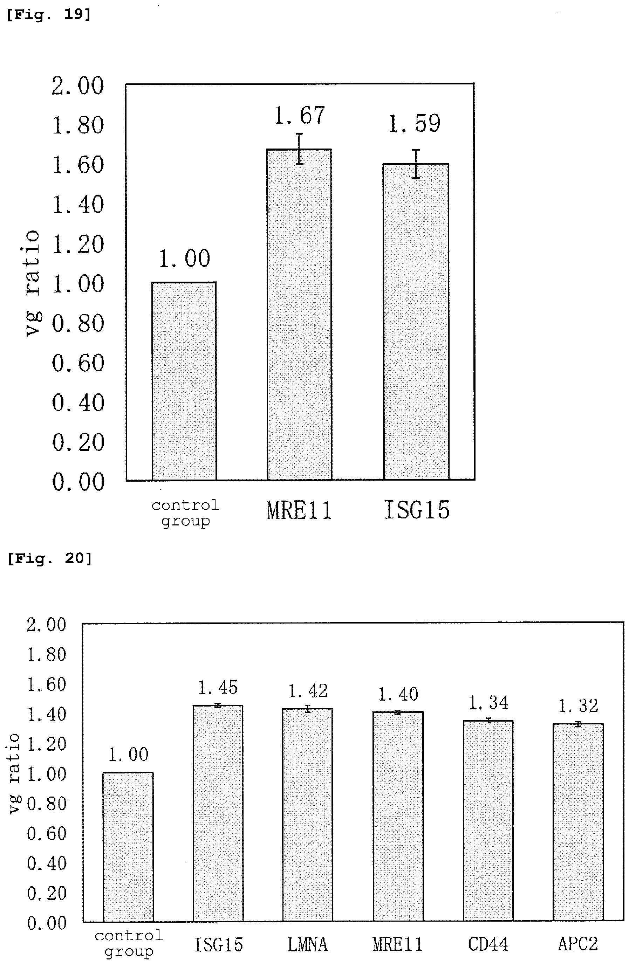

   Fig. 19 is a graph evaluating the total amount of AAV vector genomes when AAV2 vectors, which were produced by cells in which MRE11 or ISG15 was knocked down during AAV2 vector production, were sampled separately into a cell fraction and a supernatant fraction (indicated by ratio of AAV vector production amount per unit area with respect to control group).
[Fig. 20]
   Fig. 20 is a graph evaluating the amount of AAV vector genomes when AAV2 vectors, which were produced by cells in which ISG15, LMNA, MRE11, CD44, or APC2 was knocked down during AAV2 vector production, were extracted with surfactant (indicated by ratio of AAV vector production amount per unit area with respect to control group).
[Fig. 21]

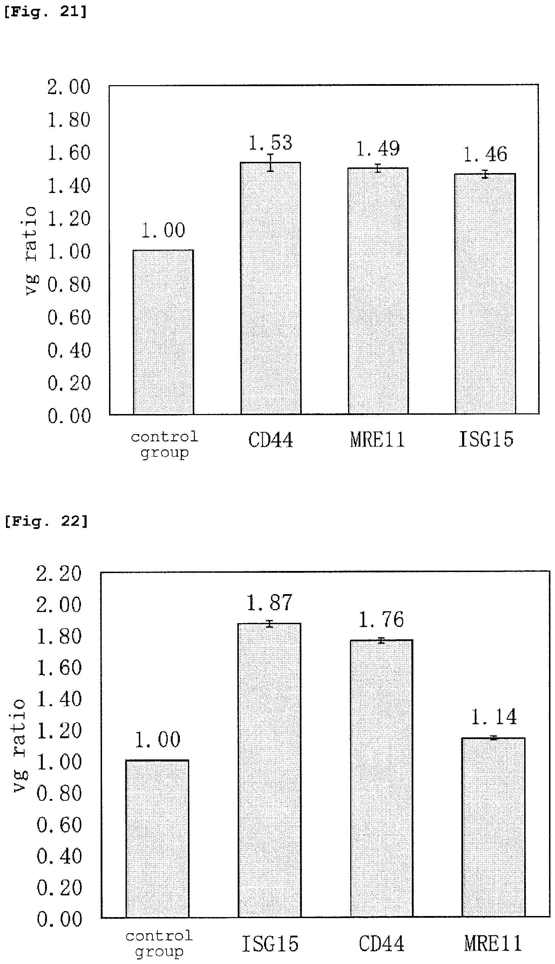

   Fig. 21 is a graph evaluating the amount of AAV vector genomes when AAV1 vectors, which were produced by cells in which CD44, MRE11, or ISG15 was knocked down during AAV1 vector production, were extracted with surfactant (indicated by ratio of AAV vector production amount per unit area with respect to control group).
[Fig. 22]
   Fig. 22 is a graph evaluating the amount of AAV vector genomes when AAV6 vectors, which were produced by cells in which ISG15, CD44, or MRE11 was knocked down during AAV6 vector production, were extracted with surfactant (indicated by ratio of AAV vector production amount per unit area with respect to control group).
[Fig. 23]

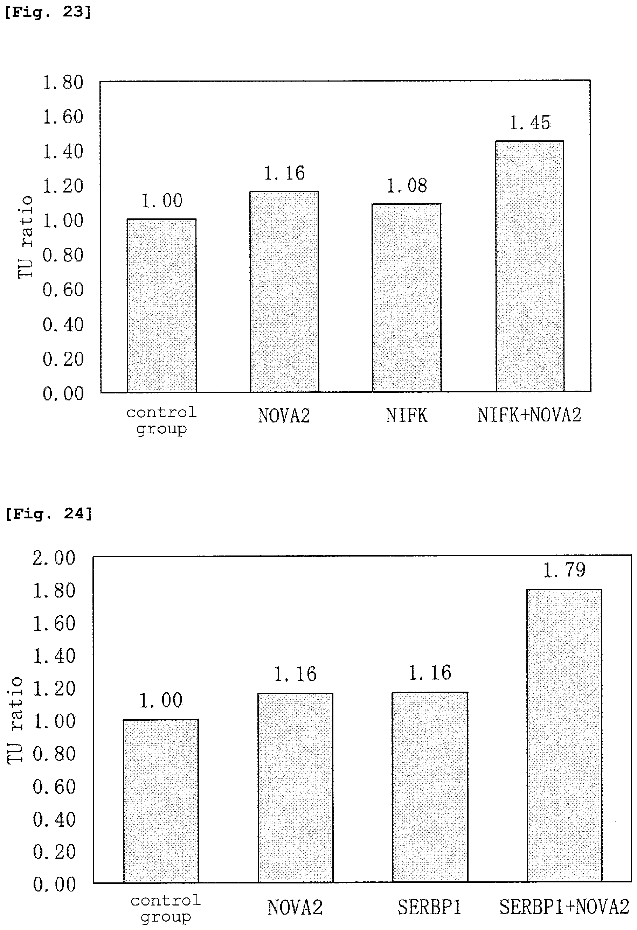

   Fig. 23 is a graph evaluating the gene transfer efficiency when AAV2 vectors, which were produced by cells that transiently expressed NOVA2 alone, NIFK alone, or NOVA2 and NIFK simultaneously during AAV2 vector production, were extracted with surfactant (indicated by ratio of AAV vector production amount per unit area with respect to control group).
[Fig. 24]
   Fig. 24 is a graph evaluating the gene transfer efficiency when AAV2 vectors, which were produced by cells that transiently expressed NOVA2 alone, SERBP1 alone, or NOVA2 and SERBP1 simultaneously during AAV2 vector production, were extracted with surfactant (indicated by ratio of AAV vector production amount per unit area with respect to control group).
[Fig. 25]

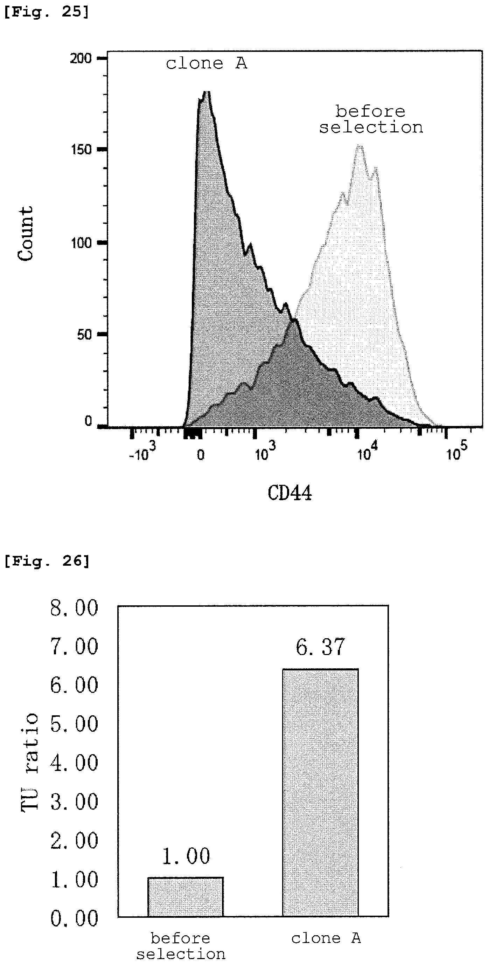

   Fig. 25 is a graph comparing the expression level of CD44 on the cell surface of clone A recovered by single-cell cloning using CD44 as a surface marker, and the cell population before selection.
[Fig. 26]
   Fig. 26 is a graph evaluating the gene transfer efficiency when AAV2 vector was produced using clone A recovered by single-cell cloning using CD44 as a surface marker, and the cell population before selection (indicated by ratio of GFP positive rate with respect to control group (cell population before selection)).
[Fig. 27]

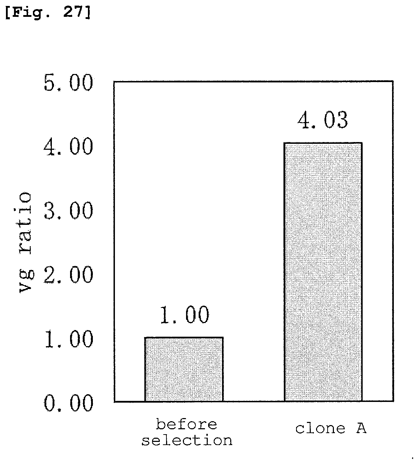

   Fig. 27 is a graph evaluating the amount of AAV vector genome when AAV2 vector was produced using clone A recovered by single-cell cloning using CD44 as a surface marker, and the cell population before selection (indicated by ratio of AAV vector production amount per unit area with respect to control group (cell population before selection)).

### [Description of Embodiments]

The present invention is described below. Unless otherwise specified, the terms used in the present specification have meanings generally used in the pertinent field.

### 1. Viral vector-producing cell and production method thereof

As cells capable of producing viral vectors (hereinafter also referred to as virus-producing cells, virus-production cells, producing cells, or production cells in the present specification), two types of 1) transiently producing cells and 2) constitutively producing cells are mainly known. Taking retrovirus-producing cells as an example, the transient producing cell is a cell that has acquired the ability to produce retroviruses by transiently introducing, into any cells, a nucleic acid construct carrying nucleic acids encoding proteins required for viral vector production (i.e., viral particle formation) and a nucleic acid construct carrying nucleic acids that supply RNA to be encapsulated in viral particles. The constitutively producing cell is a cell that has acquired the ability to produce retroviruses by introducing a retroviral vector plasmid or a retroviral vector into cells in which a nucleic acid encoding a protein essential for viral particle formation has been introduced in advance into the chromosome, and which constantly have the ability to produce the aforementioned protein.

The viral vector-producing cell of the present invention is preferably a cell with an enhanced ability to produce a viral vector, which is obtained by regulating the expression of a protein derived from the viral vector-producing cell based on comparison with the expression in the viral vector-producing cell before the regulation of the expression of the above-mentioned protein (hereinafter also to be referred to as the cell before regulation), and more preferably a cell with an enhanced ability to produce a viral vector, which is obtained by regulating the expression of a protein derived from the viral vector-producing cell as compared with the expression in HEK293 cell (Accession number ATCC CRL-1573). It may be a transiently producing cell or a constitutive producing cell, preferably a transiently producing cell.

In the present invention, "improved ability to produce vector" means that the ability to produce a vector (viral vector here) is improved compared with the cells before regulation. The degree of improvement in the production ability is not particularly limited as long as the difference is significant. The "vector production ability" may be qualitative or quantitative. For example, a qualitative improvement in vector production ability means that the ability to produce viral vectors (viral particles) with high infectivity is improved compared with that of cells before regulation. A quantitative improvement means that the amount of viral vectors (viral particles) produced has increased compared with that of cells before regulation. A qualitative improvement can be evaluated, for example, by measuring the ability of the produced viral vectors (viral particles) to infect cells (gene transfer efficiency). The ability of the produced viral vectors (viral particles) to infect cells can also be expressed as an infection titer. The gene transfer efficiency of the viral vector produced in the viral vector-producing cells of the present invention is preferably increased as compared with the gene transfer efficiency of the viral vector produced in the cells before regulation, and more preferably increased as compared with the gene transfer efficiency of the viral vector produced in HEK293 cell (Accession number ATCC CRL1573). The gene transfer efficiency of the viral vector produced in the viral vector-producing cells of the present invention means an increase of preferably 1.01 times or more, more preferably 1.02 times or more, further preferably 1.05 times or more, particularly preferably 1.1 times or more, most preferably 1.15 times or more, as compared with the gene transfer efficiency of the viral vector produced in the cells before regulation. In addition, the gene transfer efficiency of the viral vector produced in the viral vector-producing cells of the present invention means an increase of preferably 1.01 times or more, more preferably 1.02 times or more, further preferably 1.05 times or more, particularly preferably 1.1 times or more, most preferably 1.15 times or more, as compared with the gene transfer efficiency of the viral vector produced in HEK293 cell (Accession number ATCC CRL1573). Quantitative improvement can be evaluated by measuring the number of genomes (genome titer) obtained from viral vectors (viral particles) produced from the viral vector-producing cells. The genome titer of the viral vector produced in the viral vector-producing cells of the present invention is preferably increased as compared with the genome titer of the viral vector produced in the cells before regulation, and more preferably increased as compared with the genome titer of the viral vector produced in HEK293 cell (Accession number ATCC CRL1573). The genome titer of the viral vector produced in the viral vector-producing cells of the present invention means an increase of preferably 1.01 times or more, more preferably 1.02 times or more, further preferably 1.05 times or more, particularly preferably 1.1 times or more, most preferably 1.15 times or more, as compared with the genome titer of the viral vector produced in the cells before regulation. In addition, the genome titer of the viral vector produced in the viral vector-producing cells of the present invention means an increase of preferably 1.01 times or more, more preferably 1.02 times or more, further preferably 1.05 times or more, particularly preferably 1.1 times or more, most preferably 1.15 times or more, as compared with the genome titer of the viral vector produced in HEK293 cell (Accession number ATCC CRL1573) . Examples of a method for measuring the genome titer include a method of assaying, by the PCR method, the copy number of viral genome in a viral vector-containing sample in which nucleic acid impurities have been degraded by a nuclease treatment followed by a protease treatment. Examples of a method for measuring the gene transfer efficiency of a viral vector include methods such as infecting appropriate target cells with serial dilutions of a viral vector-containing sample, followed by detection of the expression of the transgene and changes in cell shape (cytopathicity), and measurement of the copy number of provirus introduced into cells, and the like. More specifically, the methods practiced in the below-mentioned Example can be mentioned.

In the present invention, the viruses from which the viral vector is derived include, but are not limited to, viruses having an envelope such as retroviruses, lentiviruses, Sendai virus, herpes viruses, and the like, viruses free of an envelope (hereinafter non-enveloped virus) such as adenoviruses, AAV, and the like, and the like. The envelope is formed when the virus buds by penetrating membranes such as the nucleus, endoplasmic reticulum, Golgi apparatus, plasma membrane, and cell membrane, generally accompanies host-derived proteins or viral proteins expressed on the host cell membrane, and plays an important role in infecting target cells.

Specifically, DNA viruses such as adenoviruses, parvovirus, papovaviruses, human papillomaviruses, and the like, and RNA viruses such as rotaviruses, coxsackieviruses, enteroviruses, sapoviruses, noroviruses, polioviruses, echoviruses, type A hepatitis virus, type E hepatitis virus, rhinoviruses, astroviruses, and the like can be mentioned. Retroviridae virus, Adenoviridae virus, and Parvoviridae virus are more preferred, and Parvoviridae adeno-associated virus (AAV) is particularly preferred. AAV has a regular icosahedral outer shell (capsid) without an envelope and a linear singlestranded DNA inside the shell. The capsid has three capsid proteins (VP1, VP2, and VP3). In the present specification, AAV includes wild-type virus and derivatives thereof, and includes all serotypes and clades unless otherwise specified. While there are various reports on AAV serotypes, at least 15 serotypes of AAV1, AAV2, AAV3a, AAV3b, AAV4, AAV5, AAV6, AAV7, AAV8, AAV9, AAV10, AAVrh.10, AAV11, AAV12, and AAV13 are known as the serotypes of AAV that infect humans.

The "viral particle" means a particle constituted of a shell of a capsid protein. Furthermore, in the present invention, the "viral particle" includes not only those containing a viral genome (nucleic acid form), but also hollow particles that are virus-like particles constituted only of capsid proteins not containing a viral genome.

In the present invention, the viral vector includes both the above-mentioned viral particles and the viral genome (nucleic acid form) contained in the viral particles. In the case of AAV, for example, a recombinant AAV (rAAV) vector means either the rAAV particle or the viral genomic DNA present in the rAAV particle.

In the present invention, the cell from which the viral vector-producing cell is derived is not particularly limited as long as the desired virus can proliferate. Cells into which a part of the gene required for the production of viral vector has been introduced are preferred, and packaging cells that do not produce viral vectors by themselves are preferred. Examples include one derived from eukaryotic cells and preferably HEK293 cells, HEK293T cell, HEK293F cells, HEK293FT cells, G3T-hi cells, Sf9 cells, commercially available cell lines for virus production, AAV293 cells, and the like, which have high transfection efficiency. HEK293 cells are preferred. In addition, for example, the aforementioned HEK293 cells and the like constitutively express adenovirus E1 protein. Such cells that are modified to transiently or constitutively express one or some of the proteins required for rAAV may also be used.

In one embodiment, examples of the element required for the production of rAAV include (A) AAV-derived Rep protein, Cap protein, (B) adenovirus-derived elements, for example, E1a, E1b, E2, E4, VARNA genes, and the like. The form of these nucleic acids is not limited, and they can be introduced into cells to be used as one or more nucleic acid constructs that can supply each element in the cells and are loaded into plasmids or viral vectors.

In the viral vector-producing cell of the present invention, the expression of the protein derived from the viral vector-producing cell is preferably regulated as compared with the expression in the cell before regulation, and more preferably regulated as compared with the expression in HEK293 cell (Accession number ATCC CRL1573). The present inventors have found that the vector-producing ability is improved by regulating the expression of such protein. In the present invention, "expression of protein is regulated" means "expression of protein is increased " or "expression of protein is decreased", preferably means "increased 1.5 times or more at the time of viral vector production, as compared with that in the cell before regulation" or "decreased not less than 10% at the time of viral vector production, as compared with that in the cell before regulation", more preferably means "increased 1.5 times or more at the time of viral vector production, as compared with that in HEK293 cell (Accession number ATCC CRL1573)" or "decreased not less than 10% at the time of viral vector production, as compared with that in HEK293 cell (Accession number ATCC CRL1573)". In addition, "expression of protein is regulated" may indicate "activity of protein is regulated". Furthermore, the expression may be regulated in two or more types of protein.

The protein whose expression should be regulated in order to improve the vector-producing ability is not particularly limited as long as the desired effect is obtained. Regulation of expression means 1.5 times or more, preferably 2.0 times or more, 3.0 times or more, 4.0 times or more, more preferably 5.0 times or more, 6.0 times or more, 7.0 times or more, further preferably 8.0 times or more, 9.0 times or more, most preferably 10.0 times or more, increase, or not less than 10%, preferably not less than 20%, more preferably not less than 30%, of decrease, as compared with that in the cell before regulation. The regulation of expression may refer to 1.5 times or more, preferably 2.0 times or more, 3.0 times or more, 4.0 times or more, more preferably 5.0 times or more, 6.0 times or more, 7.0 times or more, further preferably 8.0 times or more, 9.0 times or more, most preferably 10.0 times or more, increase, or not less than 10%, preferably not less than 20%, more preferably not less than 30%, of decrease, as compared with the expression in HEK293 cell (Accession number ATCC CRL1573).

The protein showing increased expression as compared with expression in cells before regulation or HEK293 cells (Accession number ATCC CRL1573) at the time of viral vector production includes, for example, the following proteins (see also the below-mentioned Table 1).
phosphatidylinositol 4-phosphate 3-kinase C2 domain-containing subunit alpha
Protein FAM162A
Ubiquitin-conjugating enzyme E2 C
Guanine nucleotide-binding protein-like 3
Asparagine synthetase [glutamine-hydrolyzing]
MKI67 FHA domain-interacting nucleolar phosphoprotein
Programmed cell death protein 4
Probable ATP-dependent RNA helicase DDX47
Nucleolar GTP-binding protein 1
pre-rRNA processing protein FTSJ3
Probable ATP-dependent RNA helicase ddx49
Nucleolar GTP-binding protein 2
RNA cytidine acetyltransferase
Translation machinery-associated protein 7
ubiquitin-like protein 5
DNA (cytosine-5)-methyltransferase 1
Plasminogen activator inhibitor 1 RNA-binding protein
RNA-binding protein Nova-2
DNA-directed RNA polymerase II subunit RPB1

The protein showing decreased expression as compared with expression in cells before regulation or HEK293 cells (Accession number ATCC CRL1573) at the time of viral vector production includes, for example, the following proteins (see also the below-mentioned Table 2).
Ubiquitin-like protein ISG15
CD44 antigen
Prelamin-A/C
Lysosomal acid phosphatase
hydroxymethylglutaryl-CoA synthase, cytoplasmic
Double-strand break repair protein MRE11

The protein showing increased expression as compared with expression in cells before regulation or HEK293 cells (Accession number ATCC CRL1573) at the time of viral vector production preferably includes phosphatidylinositol 4-phosphate 3-kinase C2 domain-containing subunit alpha (PIK3C2A), MKI67 FHA domain-interacting nucleolar phosphoprotein (NIFK), Ubiquitin-conjugating enzyme E2 C (UBE2C), Asparagine synthetase [glutamine-hydrolyzing] (ASNS), Nucleolar GTP-binding protein 1 (GTPBP4), pre-rRNA processing protein FTSJ3 (FTSJ3), Probable ATP-dependent RNA helicase ddx49 (DDX49), Nucleolar GTP-binding protein 2 (GNL2), Plasminogen activator inhibitor 1 RNA-binding protein (SERBP1), and RNA-binding protein Nova-2 (NOVA2). It is more preferably NIFK, FTSJ3, DDX49, SERBP1, or NOVA2. It is further preferably FTSJ3 or NOVA2.

PIK3C2A is one of the PI3 kinase family and is a protein involved in signal transduction in cell proliferation, intracellular protein transport, and the like.

NIFK is a protein that binds to RNA and contributes to mitosis and acceleration of the cell cycle, though its function is largely not elucidated.

UBE2C is known as an enzyme that accepts ubiquitin from E1 and catalyzes covalent binding to other proteins.

ASNS is an asparagine synthase that synthesizes L-asparagine and L-glutamate from L-aspartate and L-glutamine in an ATP-dependent manner.

GTPBP4 is one type of GTPase and is known to be highly expressed in various cancer cells and proliferative cells.

FTSJ3 is one type of methyltransferase and has recently been reported to be involved in 2'-O-methylation of RNA, which is a molecular marker for the cellular innate immune system to distinguish between endogenous and exogenous mRNAs.

DDX49 is an RNA helicase that has been suggested to be involved in viral infections and the like, and its detailed function has not been elucidated.

GNL2 is a GTPase that is involved in the transport and maturation of 60S ribosomal subunit, and its cell proliferation-promoting effect and the like have also been reported.

SERBP1 has been suggested to be involved in the stability regulation of mRNA.

NOVA2 binds to single strand RNA and is suggested to be involved in RNA splicing and metabolism in nerve cells and the like.

The protein showing decreased expression as compared with expression in cells before regulation or HEK293 cells-(Accession number ATCC CRL1573) at the time of viral vector production preferably includes, for example, Ubiquitin-like protein ISG15 (ISG15), Double-strand break repair protein MRE11 (MRE11), and CD44 antigen (CD44).

ISG15 is a ubiquitin-like molecule considered to play an important role in the innate immune response to viral infection.

MRE11 is a constituent element of the MRN complex that plays a major role in repair of double-strand breaks, recombination, maintaining telomere, and the like.

CD44 is known as a hyaluronic acid receptor, and is a protein involved in cell aggregation, matrix retention around cells, and the like.

The ability to produce viral vectors is enhanced as compared with the expression in the cells before regulation and HEK293 cell (Accession number ATCC CRL1573), by regulating the expression of two or more kinds of proteins at the time of viral vector production. A combination of two or more kinds of proteins includes, for example, RNA-binding protein Nova-2 (NOVA2) and MKI67 FHA domain-interacting nucleolar phosphoprotein (NIFK), and RNA-binding protein Nova-2 (NOVA2) and Plasminogen activator inhibitor 1 RNA-binding protein (SERBP1).

The method for regulating the expression of protein is not particularly limited as long as a desired effect, that is, an increase or decrease in the expression of protein derived from the viral vector-producing cells, preferably 1.5 times or more increase or decrease by not less than 10%, as compared with the expression in the cells before regulation or HEK293 cell (Accession number ATCC CRL1573), can be afforded. For example, a method for inducing protein expression in viral vector-producing cells by adding additives (embodiment 1), a method for suppressing (inhibiting) protein expression in viral vector-producing cells by adding additives (embodiment 2), a method for introducing an exogenous plasmid that induces expression of a protein into viral vector-producing cells to increase its expression level (embodiment 3), and a method for introducing an exogenous plasmid that suppresses (inhibits) expression of a protein into viral vector-producing cells to decrease its expression level (embodiment 4) can be mentioned. Examples of the method for measuring the protein expression level include, but are not limited to, Western blotting and ELISA.

Examples of the additives for inducing protein expression in viral vector-producing cells used in embodiment 1 include proteins desired to show enhanced expression in viral vector-producing cells and genes encoding same (e.g., mRNA). As the gene, at least one type of protein listed in Table 1 can be mentioned. Gene introduction can be performed by methods generally used in the art for nucleic acid introduction (e.g., calcium phosphate method, lipofection method, DEAE dextran method, polyethyleneimine method, electroporation method, introduction by retroviral vector). A case in which an exogenous plasmid, which is a gene encoding a protein whose expression in viral vector-producing cells is desired to be enhanced, is used as the additive is described in detail as embodiment 3.

The additive may also be an activator capable of inducing expression of the protein (e.g., a low-molecular-weight compound that acts allosterically to increase the enzyme activity).

Additives for suppressing (inhibiting) protein expression in viral vector-producing cells used in embodiment 2 include those capable of suppressing intracellular expression of proteins whose expression is desired to be reduced in viral vector-producing cells. Specifically, nucleic acids (e.g., antisense nucleic acid, siRNA, shRNA) capable of suppressing (inhibiting) the protein expression can be mentioned. For example, nucleic acids that suppress (inhibit) the expression of at least one type of proteins listed in Table 2 can be mentioned. Nucleic acids can be introduced by methods generally used in the art for nucleic acid introduction (e.g., calcium phosphate method, lipofection method, DEAE dextran method, polyethyleneimine method, electroporation method, introduction by retroviral vector). A case in which an exogenous plasmid, which is a nucleic acid capable of suppressing (inhibiting) the expression of a protein whose expression in viral vector-producing cells is desired to be decreased, is used as the additive is described in detail as embodiment 4.

The additive may also be an inhibitor (e.g., low-molecular-weight compound, protein) capable of decreasing the expression of the protein.

The exogenous plasmid that induces the expression of the protein used in embodiment 3 (hereinafter to be also referred to as exogenous inducing plasmid) is a plasmid capable of expressing a protein exogenous to the viral vector-producing cells, for example, at least one of the proteins listed in Table 1, preferably at least one selected from the group consisting of PIK3C2A, UBE2C, ASNS, NIFK, PDCD4, DDX47, GTPBP4, FTSJ3, DDX49, GNL2, NAT10, UBL5, SERBP1, and NOVA21, more preferably at least one selected from the group consisting of PIK3C2A, NIFK, UBE2C, ASNS, GTPBP4, FTSJ3, DDX49, GNL2, SERBP1, and NOVA2. It may be a plasmid capable of expressing a combination of NIFK and NOVA2, or SERBP1 and NOVA2. Each plasmid can be prepared based on the genetic information of each protein listed in Table 1.

The exogenous plasmid that suppresses (inhibits) the expression of the protein used in embodiment 4 (hereinafter exogenous suppressing plasmid) is a plasmid capable of suppressing (inhibiting) a protein exogenous to the viral vector-producing cells, for example, at least one of the proteins listed in Table 2, preferably at least one selected from the group consisting of ISG15, CD44, LMNA, ACP2, and MRE11, more preferably at least one selected from the group consisting of ISG15, MRE11, and CD44. Suppression (inhibition) of expression may be performed by knocking out or knocking down a gene. Gene knockdown refers to reducing the transcription level of a specific gene or inhibiting translation from a specific gene. Different from gene knockout, which destroys the gene itself, knockdown greatly attenuates, but not completely eliminates, the function of the gene. As methods for knocking out a gene, methods well known in the art can be used, such as a method for disrupting the gene by using a vector (targeting vector) that causes homologous recombination at any position of the target gene (gene targeting method), and a method for inserting a trap vector (a reporter gene without a promoter) into any position of the target gene to disrupt and render the gene non-functional. As methods for knocking down a gene, for example, methods well known in the art can be used, such as an antisense method in which RNA corresponding to the antisense strand of mRNA is introduced into cells, an RNAi method using siRNA, shRNA, microRNA, and the like. For example, as a method for knocking down the expression of ISG15, introduction of siRNA prepared based on the genetic information of Uniprot ID: P05161 can be mentioned. As a method for knocking down the expression of MRE11, introduction of siRNA prepared based on the genetic information of Uniprot ID: P05161 can be mentioned. As a method for knocking down the expression of CD44, introduction of siRNA prepared based on the genetic information of Uniprot ID: P16070 can be mentioned. Knockdown efficiency can be confirmed by measuring changes in mRNA levels and protein expression levels. Methods for measuring mRNA levels include, but are not limited to, real-time quantitative PCR (qPCR) and Northern blotting. Methods for measuring protein expression levels include, but are not limited to, Western blotting and ELISA.

In the present invention, the knockdown efficiency is not particularly limited as long as the viral vector productivity can be improved in the obtained viral vector-producing cells. For example, the efficiency is about 30%, about 35%, about 40%, about 45%, about 50%, about 55%, about 60%, about 65%, about 70%, about 75%, about 80%, about 85%, about 90%, about 95%, about 96%, about 97%, about 98%, or about 99%.

The exogenous inducing plasmid and exogenous suppressing plasmid are also to be collectively referred to as exogenous regulation plasmid.

The methods of plasmid introduction are exemplified by transient introduction methods and constitutive introduction methods. It can be loaded in the same plasmid as the plasmid for viral vector production or in a separate plasmid, and preferably loaded in a separate plasmid.

The method of transient introduction is not particularly limited, and known transient introduction methods such as calcium phosphate method, lipofection method, DEAE dextran method, polyethyleneimine method, electroporation method, and the like can be used. Moreover, a commercially available reagent may also be used.

The method of constitutive introduction is not particularly limited, and known constitutive introduction methods such as a method using a retroviral vector, and a method of introducing by a method similar to the transient introduction method of a plasmid and selecting cells that have integrated into the chromosome, and the like can be used. In a method using a retroviral vector, a commercially available reagent may also be used.

The viral vector-producing cell of the present invention can be produced in the same manner as in the methods generally practiced in the art, except that it includes a step of regulating the expression of a protein derived from a viral vector-producing cell as compared with the expression in cells before regulation or HEK293 cells (Accession number ATCC CRL1573).

The viral vector-producing cell of the present invention can be produced by introducing a nucleic acid to be encapsulated in a viral particle into a cell having the ability to produce a viral vector, which is a material, and performing a step of regulating the expression of a protein derived from a viral vector-producing cell as compared with the expression in cells before regulation or HEK293 cells (Accession number ATCC CRL1573). For example, in the case of AAV, the aforementioned nucleic acid to be encapsulated in the viral particle is composed of an AAV-derived ITR sequence and a nucleic acid desired to be loaded into the AAV vector. The aforementioned nucleic acid desired to be loaded into the AAV vector is exemplified by nucleic acids encoding any exogenous gene, such as polypeptides (enzyme, growth factor, cytokine, receptor, structural protein, and the like), antisense RNA, ribozyme, decoy, RNA that causes RNA interference, and the like. Appropriate promoters, enhancers, terminators, and other transcriptional regulation elements may be inserted into the aforementioned nucleic acid in order to control the expression of the aforementioned exogenous gene. The nucleic acids to be encapsulated in viral particles can be introduced in the form of plasmids into cells.

The step of regulating the expression of a protein derived from a viral vector-producing cell as compared with the expression in cells before regulation or HEK293 cells (Accession number ATCC CRL1573) is a step aiming at enhancing or reducing the expression of the protein, and includes, for example, the methods of embodiment 1 to embodiment 4 exemplified as methods for regulating the expression of protein mentioned above.

The thus-obtained viral vector-producing cells are cells with enhanced ability to produce viral vectors. Therefore, the present invention provides a method for producing a viral vector-producing cell with improved viral vector-producing ability. The method is also a method for improving the viral vector-producing ability of viral vector-producing cells.

That is, the present invention provides a method for enhancing the production amount of a viral vector in viral vector-producing cells, including regulating the expression of a protein derived from a viral vector-producing cell as compared with the expression in cells before regulation or HEK293 cells (Accession number ATCC CRL1573).

By regulating (enhancing or reducing) the expression of a protein derived from a viral vector-producing cell as compared with the expression in cells before regulation or HEK293 cells (Accession number ATCC CRL1573), in cells capable of proliferating the desired virus, preferably cells into which a part of the gene necessary for the production of the viral vector has been introduced, the virus production ability of the viral vector-producing cells can be improved (each term is as defined above).

Also, the present invention provides a method for selecting viral vector-producing cells with improved viral vector-producing ability, including selecting cells in which the expression of a protein derived from a viral vector-producing cell is regulated as compared with the expression in cells before regulation or HEK293 cells (Accession number ATCC CRL1573).

The cells in which the expression of a protein derived from a viral vector-producing cell is regulated as compared with the expression in cells before regulation or HEK293 cells (Accession number ATCC CRL1573), in cells capable of proliferating the desired virus, preferably cells into which a part of the gene necessary for the production of the viral vector has been introduced, are viral vector-producing cells with improved viral vector-producing ability, and such viral vector-producing cells can be selected according to the present invention (each term is as defined above). For example, viral vector-producing cells with improved viral vector-producing ability can be obtained by selecting cells, in which the expression of CD44 antigen is reduced as compared with the expression in cells before regulation or HEK293 cells (Accession number ATCC CRL1573), from a certain cell population.

### 2. Production method of viral vector

The present invention relates to a method for producing a viral vector, particularly to a method for efficiently producing same. The method for producing a viral vector of the present invention can be performed in the same manner as in the methods generally practiced in the art except that the viral vector-producing cell of the present invention, namely, viral vector-producing cell with improved ability to produce vector, in which the expression of a protein derived from a viral vector-producing cell is preferably regulated as compared with the expression in cells before regulation and more preferably regulated as compared with the expression in HEK293 cells (Accession number ATCC CRL1573), is used as the packaging cell.

The production of the viral vector of the present invention is performed by culturing viral vector-producing cells obtained by a method including a step of transiently or constitutively introducing into the viral vector-producing cell of the present invention a nucleic acid that supplies essential elements for viral particle formation. The method for transiently or constitutively introducing a nucleic acid is not particularly limited and, for example, the known transient or constitutive introduction method described above as the plasmid introduction method may also be used.

Culture of the cells can be performed under known culture conditions. Examples include, but are not limited to, culturing at temperature 30 to 37°C, humidity 95%RH, CO₂ concentration 5 to 10% (v/v). Temperature, humidity, and CO₂ concentration outside the aforementioned ranges may be used as long as the proliferation of viral vector-producing cells and the production of viral particles can be achieved. The culture period is not particularly limited, and is, for example, 12 to 150 hr, preferably 48 to 120 hr. The medium used for culturing the viral vector-producing cells may contain components necessary for culturing the cells. For example, basic synthetic medium such as DMEM, IMDM, DMEM:F-12 and the like, and these basal synthetic media added as necessary with fetal bovine serum, growth factors, peptides, or increased amount of amino acids can be mentioned.

Viral vectors can be collected from viral vector-producing cells by methods generally practiced in the art. Examples include mechanical stirring, ultrasonic disruption, freeze-thawing, solution extraction methods, chemical methods for appropriately adjusting the pH and salt concentration of the solution to be extracted, and methods in which these are combined.

Viral vectors produced using the viral vector-producing cell of the present invention are not particularly limited, and examples thereof include recombinant viral vectors derived from existing serotypes or wild-type viruses newly obtained from nature. In the case of AAV, a recombinant AAV vector based on the AAV serotype desired to be used can be produced by selecting appropriate materials, such as nucleic acid encoding Rep protein and nucleic acid encoding Cap protein, when producing the cells of the present invention.

### 3. Kit (kit for producing viral vector)

The present invention provides a kit for producing a viral vector, containing the viral vector-producing cell of the present invention or viral vector-producing cells obtained by the production method of the viral vector-producing cell of the present invention. This kit may contain nucleic acids that supply proteins necessary for viral particle formation of viral vector (e.g., in the case of AAV; nucleic acid encoding Rep protein, nucleic acid encoding Cap protein, and nucleic acid encoding adenovirus-derived protein).

As a kit for performing the production of the viral vector-producing cell of the present invention and the production of a viral vector in a series of steps, a kit containing (i) a viral vector-producing cell prior to regulation, (ii) an exogenous regulation plasmid (exogenous inducing plasmid or exogenous suppressing plasmid), and (iii) a nucleic acid that supplies proteins necessary for viral particle formation of the viral vector can be mentioned. An exogenous regulation plasmid is introduced into viral vector-producing cells before regulation to obtain viral vector-producing cells with enhanced viral vector-producing ability, and the cells are used to produce viral vectors.

The present invention is described in detail below using examples, but the present invention is not limited in any way. Reagents and materials to be used are commercially available unless otherwise specified, or can be prepared from known literature and the like. In addition, those skilled in the art understand that other substances having similar effects and actions can be used alternatively.

### [Example]

### Example 1

### (1) Preparation of viral vector-producing cell pellets and nonproducing cell pellets

HEK293 cells (Accession number ATCC CRL1573) suspended in DMEM (Sigma Ltd.) containing 10% FBS (Gibco) and 1% MEM non-essential amino acid solution (100x) (Nacalai Tesque) and HEK293T cells (manufactured by Takara Bio Inc., product number 632273) suspended in DMEM containing 10% FBS were seeded in a T225 flask (Corning Incorporated). Thereafter, the culture was continued in a CO₂ incubator at 37°C, and the cells were confirmed to be approximately 70-80% confluent.

Plasmids (i) to (iii) were transfected into HEK293 cells and HEK293T cells of the AAV vector production group.
(i) Plasmid encoding AAV2 Rep protein and Cap protein (Takara Bio Inc., pRC2-mi342 Vector)
(ii) Plasmid containing adenovirus E2A sequence, VA sequence, E4 sequence (Takara Bio Inc., pHelper Vector)
(iii) Plasmid containing expression cassette of fluorescent protein GFP between two ITRs of AAV2 (CELL BIOLABS, pAAV-GFP)

Only a transfection reagent and DMEM in the same amount as in the AAV vector production group were added to HEK293 cells and HEK293T cells in the control group.

The cells were cultured in a CO₂ incubator at 37°C and, after 24 hr of transfection, the medium in the HEK293 cell culture flask was changed with DMEM containing 2% FBS and 1% MEM non-essential amino acid solution (100×), and the medium in the HEK293T cells culture flask was changed with DMEM containing 2% FBS.

The cells were cultured in a CO₂ incubator at 37°C, and 48 hr after transfection, 0.5M EDTA (pH 8.0) (Sigma Ltd.) was added at 1/80 volume. After allowing to stand at room temperature for 10 min, detached cells were collected in a centrifugation tube. After centrifugation at 400×g, 4°C for 5 min, the supernatant was removed and the cell pellet was washed with DPBS (WAKO). A cell suspension of 2×10⁶ cells was transferred to a tube, centrifuged at 400×g, 4°C for 5 min, the supernatant was removed, and the cell pellet was stored at -80°C until use.

### (2) Identification of host cell protein by LC-MS/MS measurement

100 µL of lysis buffer (8 M Urea (FUJIFILM Wako Pure Chemical Corporation), 50 mM TEAB (Thermo Fisher Scientific, Inc.), pH 8.0) was added to the cell pellet consisting of 2×10⁶ cells in Example 1-(1).

After adding Endonuclease (KANEKA CORPORATION) to reduce the viscosity, the mixture was centrifuged at 16,000×g, 4°C for 10 min, and the supernatant was collected. Thereafter, the total amount of protein contained in the collected solution was measured using Pierce BCA Protein Assay Kit (Thermo Fisher Scientific, Inc.) according to the manufacturer's protocol. Based on the measurement results, 100 µL of a solution with a total protein concentration of 1 µg/µL was prepared, 5 µL of 200 mM TCEP (Thermo Fisher Scientific, Inc.) was added, and the mixture was reacted at 55°C for 1 hr.

5 µL of 375 mM iodoacetaminde (Thermo Fisher Scientific, Inc.) was added, and the mixture was reacted for 30 min at room temperature in the dark. 600 µL of acetone (Kanto Chemical Co., Inc.) was added, and the mixture was allowed to stand overnight at -30°C to cause protein precipitation. After centrifugation at 8,000×g, 4°C for 10 min, the supernatant was removed, the pellet was dried and then dissolved in 1 mL of 50 mM TEAB. 2.5 µg of trypsin (Promega KK) was added, and digested overnight at 37°C. The pH of the solution was adjusting to about 3 to terminate the digestion reaction, and samples corresponding to about 50 µg of total protein were collected and adjusted to 100 µL with 100 mM TEAB.

Using TMT-10plex (Thermo Fisher Scientific, Inc.), samples for LC-MS/MS measurement were prepared according to the manufacturer's protocol. LC-MS/MS was performed using a system connecting Easy-nLC1200 (Thermo Fisher Scientific, Inc.) and Orbitrap Eclipse (Thermo Fisher Scientific, Inc.), and analyzed by Orbitrap Eclipse. Using Proteome Discoverer 2.4 (Thermo Fisher Scientific, Inc.), identification and quantify analysis of TMT-labeled samples were performed.

Four 4 different cell lysate samples (AAV vector-producing HEK293 cells, control HEK293 cells, AAV vector-producing HEK293T cells, control HEK293T cells) were subjected to LC-MS/MS analysis. The operations from Example 1-(1) to Example 1-(2) were independently performed twice, and MS analysis was performed three times for each batch sample. From the identified host cell-derived proteins were extracted proteins satisfying p<0.05, showing 1.5 times or more variation in the amount present during viral vector production as compared with non-production in HEK293 cells or HEK293T cells, and showing 1.5 times or more difference between HEK293 cells and HEK293T cells in the amount present during viral vector production. The proteins that satisfied the conditions are listed in Table 1 and Table 2.

These proteins were ranked as follows based on the quantitative presence ratio between HEK293 cells and HEK293T cells during AAV vector production.

A protein with AAV vector producing HEK293T cells/AAV vector producing HEK293 cells or AAV vector producing HEK293 cells/AAV vector producing HEK293T cells of 10.0 times or more was designated as A, a protein with 3.0 times or more and less than 10.0 times was designated as B, and a protein with 1.5 times or more and less than 3.0 times was designated as C. In Table 1, the amount of protein present in HEK293T cells during AAV vector production is higher than that in HEK293 cells during AAV vector production, and in Table 2, the amount of protein present in HEK293T cells during AAV vector production is lower than that in HEK293 cells during AAV vector production.

**[Table 1]**

| Uniprot ID | protein name | AAV vector producing HEK293T cell/AAV vector producing HEK293 cell | | | |
|---|---|---|---|---|---|
| | | test 1 | test 2 | average | rank |
| 000443 | phosphatidylinositol 4-phosphate 3-kinase C2 domain-containing subunit alpha | 25, 65 | 13, 24 | 19. 44 | A |
| Q96A26 | Protein FAM162A | 2. 43 | 12. 71 | 7.57 | B |
| 000762 | Ubiquitin-conjugating enzyme E2 C | 6.97 | 2.46 | 4. 72 | B |
| Q9BVP2 | Guanine nucleotide-binding protein-like 3 | 4.54 | 4. 35 | 4. 44 | B |
| P08243-1 | Asparagine synthetase [glutamine-hydrolyzing] | 4. 54 | 2. 97 | 3. 75 | B |
| Q9BYG3 | MKI67 FHA domain-interacting nucleolar phosphoprotein | 4.24 | 3.09 | 3.66 | B |
| Q53EL6-1 | Programmed cell death protein 4 | 4.28 | 2.29 | 3.28 | B |
| Q9HOS4 | Probable ATP-dependent RNA helicase DDX47 | 3.32 | 3. 10 | 3.21 | B |
| Q9BZE4 | Nucleolar GTP-binding protein 1 | 2.62 | 3.67 | 3.14 | B |
| QSIY81 | pre-rRNA processing protein FTSJ3 | 3. 27 | 2.82 | 3.05 | B |
| Q9Y6V7 | Probable ATP-dependent RNA helicase ddx49 | 2.44 | 3. 48 | 2.96 | C |
| Q13823 | Nucleolar GTP-binding protein 2 | 2. 79 | 3. 05 | 2.92 | C |
| Q9H0A0 | RNA cytidine acetyl transferase | 3.07 | 2. 64 | 2.85 | C |
| Q9Y2S6 | Translation machinery-associated protein 7 | 2. 85 | 2.26 | 2.55 | C |
| Q9BZL1 | ubiquitin-like protein 5 | 3.21 | 1.79 | 2.50 | C |
| P26358-1 | DNA (cytosine-5)-methyl transferase 1 | 2. 24 | 2. 60 | 2.42 | C |
| Q8NC51-1 | Plasminogen activator inhibitor 1 RNA-binding protein | 2.17 | 2, 66 | 2.41 | C |
| Q9UNW9 | RNA-binding protein Nova-2 | 2. 14 | 2.51 | 2. 32 | c |
| P24928 | DNA-directed RNA polymerase II subunit RPBI | 2. 52 | 1. 63 | 2.07 | C |

**[Table 2]**

| Uniprot ID | protein name | AAV vector producing HEK293 cell/AAV vector producing HEK293T cell | | | |
|---|---|---|---|---|---|
| | | test 1 | test 2 | average | rank |
| P05161 | Ubiquitin-like protein ISG15 | 14.29 | 21.76 | 18. 02 | A |
| P16070 | CD44 antigen | | 127.9 | 127. 9 | A |
| P02545 | Prelamin-A/C | 2. 09 | 2. 00 | 2.04 | C |
| P11117 | Lysosomal acid phosphatase | 1.93 | 2. 05 | 1.99 | C |
| Q01581 | hydroxymethylglutaryl-CoA synthase, cytoplasmic | 1.54 | 2.10 | 1.82 | C |
| P49959 | Double strand break repair protein MRE11 | 1. 62 | 1. 58 | 1.60 | C |

### Example 2

### (1) Transient expression of PIK3C2A, UBE2C, ASNS, NIFK, PDCD4, DDX47, GTPBP4, FTSJ3, DDX49, GNL2, NAT10, UBL5, SERBP1, and NOVA2 in production cell

In Table 1, phosphatidylinositol 4-phosphate 3-kinase C2 domain-containing subunit alpha is indicated as PIK3C2A, Ubiquitin-conjugating enzyme E2 C as UBE2C, Asparagine synthetase [glutamine-hydrolyzing] as ASNS, MKI67 FHA domain-interacting nucleolar phosphoprotein as NIFK, Programmed cell death protein 4 as PDCD4, Probable ATP-dependent RNA helicase DDX47 as DDX47, Nucleolar GTP-binding protein 1 as GTPBP4, pre-rRNA processing protein FTSJ3 as FTSJ3, Probable ATP-dependent RNA helicase ddx49 as DDX49, Nucleolar GTP-binding protein 2 as GNL2, RNA cytidine acetyltransferase as NAT10, ubiquitin-like protein 5 as UBL5, Plasminogen activator inhibitor 1 RNA-binding protein as SERBP1, and RNA-binding protein Nova-2 as NOVA2.

HEK293 cells (manufactured by Agilent, product number 240073) suspended in DMEM containing 10% FBS were seeded in a 6-well plate for cell culture (Corning Incorporated). Thereafter, the culture was continued in a CO₂ incubator at 37°C, and the cells were confirmed to be approximately 70-80% confluent. The culture supernatant of the cells was removed, replaced with serum-free DMEM, and plasmids (i) to (iv) were transfected. Plasmids (ii) to (iv) and empty vector were transfected in the same manner into the cells of the control group.

### (i) Plasmid encoding corresponding factor

(Vector Builder, pRP-Neo-CAG-hPIK3C2A Vector or pRP-Neo-CAG-hUBE2C Vector or pRP-Neo-CAG-hASNS Vector or pRP-Neo-CAG-hNIFK Vector or pRP-Neo-CAG-hPDCD4 Vector or pRP-Neo-CAG-hDDX47 Vector or pRP-Neo-CAG-hGTPBP4 Vector or pRP-Neo-CAG-hFTSJ3 Vector or pRP-Neo-CAG-hDDX49 Vector or pRP-Neo-CAG-hGNL2 Vector or pRP-Neo-CAG-hNAT10 Vector or pRP-Neo-CAG-hUBL5 Vector or pRP-Neo-CAG-hSERBP1 Vector or pRP-Neo-CAG-hNOVA2 Vector)

**[Table 3]**

| corresponding factor name | plasmid name |
|---|---|
| PIK3C2A | pRP-Neo-CAG-hPIK3C2A Vector |
| UBE2C | pRP-Neo-CAG-hUBE2C Vector |
| ASNS | pRP-Neo-CAG-hASNS Vector |
| NIFK | pRP-Neo-CAG-hNIFK Vector |
| PDCD4 | pRP-Neo-GAG-hPDCD4 Vector |
| DDX47 | pRP-Neo-CAG-hDDX47 Vector |
| GTPBP4 | pRP-Neo-CAG-hGTPBP4 Vector |
| FTS J3 | pRP-Noo-CAG-hFTSJ3 Vector |
| DDX49 | pRP-Neo-CAG-hDDX49 Vector |
| GNL2 | pRP-Neo-CAG-hGNL2 Vector |
| NAT10 | pRP-Neo-CAG-hNAT10 Vector |
| UBL5 | pRP-Neo-CAG-hUBL5 Vector |
| SERBP1 | pRP-Neo-CAG-hSERBP1 Vector |
| NOVA2 | pRP-Neo-CAG-hNOVA2 Vector |

### (ii) pRC2-mi342 Vector or pRC1 Vector (Takara Bio Inc.) or pRC6 Vector (Takara Bio Inc.)

**[Table 4]**

| serotype | plasmid name |
|---|---|
| AAV2 | pRC2-mi342 Vector |
| AAV1 | pRC1 Vector |
| AAV6 | pRC6 Vector |

### (iii) pHelper Vector

### (iv) pAAV-GFP

After transfection, cultured in a CO₂ incubator at 37°C for 3 days.

### (2) Sampling of supernatant fraction AAV vector

After completion of the culture in Example 2-(1), a part of the culture medium was sampled and centrifuged (1,800×g, 10 min, 4°C). The supernatant after centrifugation was collected and used as "AAV vector supernatant fraction sample". After sampling, 0.5 M EDTA (pH 8.0) was added to the culture medium remaining in the well plate at 1/80 volume of the culture medium and mixed well. After reaction at room temperature for 10 min, the cells were detached. The detached cells were collected as the solution and centrifuged (1,800×g, 10 min, 4°C). After centrifugation, the supernatant was removed to give a cell pellet.

### (3) Sampling of cell fraction AAV vector

To the cell pellet of Example 2-(2) was added 80 µL of Extraction Solution A (AAVpro (registered trademark) Extraction Solution Takara Bio Inc.), and the cells were suspended by vortexing for 15 sec. After allowing to stand at room temperature for 5 min, the cells were further suspended by vortexing for 15 sec. The cell suspension was centrifuged (9,000×g, 10 min, 4°C). The cells were further suspended by vortexing for 15 sec. After allowing to stand at room temperature for 5 min, the cells were further suspended by vortexing for 15 sec. After centrifugation (9,000×g, 10 min, 4°C), the supernatant was collected, 8 µL of Extraction Solution B (AAVpro (registered trademark) Extraction Solution Takara Bio Inc.) was added thereto and mixed well to give "AAV vector cell fraction sample".

### (4) Sampling of AAV vector with surfactant

As a sampling method of AAV vector different from Example 2-(2)(3), sampling using a surfactant was performed. Tween20 (Sigma Ltd.) was added at a 1/1000 volume of the culture medium after completion of culture in Example 2-(1), 2 mM MgCl₂ was added to a final concentration and Endonuculease (KANEKA CORPORATION) was added at a final concentration of 50U/mL and they were stirred well. The cells were lysed by incubating in a CO₂ incubator at 37°C for 2 hr, and the culture medium was transferred into a centrifuge tube and centrifuged at 1,800×g for 10 min. The supernatant after centrifugation was used as an "AAV vector surfactant extraction sample (supernatant fraction+cell fraction)".

### Example 3

### (1) MRE11, ISG15, CD44, LMNA and ACP2 knockdown in production cell

In Table 2, Ubiquitin-like protein ISG15 is indicated as ISG15, Double-strand break repair protein MRE11 as MRE11, CD44 antigen as CD44, Prelamin-A/C as LMNA, and Lysosomal acid phosphatase as ACP2.

HEK293 cells (manufactured by Agilent, product number 240073) suspended in DMEM containing 10% FBS were seeded in a 6-well plate for cell culture. Thereafter, the culture was continued in a CO₂ incubator at 37°C, and the cells were confirmed to be approximately 70-80% confluent. The culture supernatant was removed, and the medium was replaced with serum-free DMEM. Using Lipofectamine3000 (invitrogen), siRNA of (i) and plasmids of (ii) to (iv) were transfected. Plasmids (ii) to (iv) were transfected in the same manner into the cells of the control group.

### (i) siRNA for suppressing expression of corresponding factor (Sigma Ltd., si-MRE11 or si-ISG15 or si-CD44) (Thermo Fisher Scientific, Inc., LMNA Silencer or ACP2 Silencer)

**[Table 5]**

| corresponding factor name | siRNA name | sequence |
|---|---|---|
| MRE11 | si-MRE11 | GCAUCCUGGUGAGGAAUAACAAGGG (SEQ ID NO:1) |
| ISG15 | si-ISG15 | AAGAACCUGGUCCCAGAGGAG (SEQ ID NO:2) |
| CD44 | si-CD44 | GGACCAAUUACCAUAACUATT (SEQ ID NO:3) |
| LMNA | LMNA Silencer | - |
| ACP2 | APC2 Silencer | - |

### (ii) pRC2-mi342 Vector or pRC1 Vector or pRC6 Vector

**[Table 6]**

| serotype | plasmid name |
|---|---|
| AAV2 | pRC2-mi342 Vector |
| AAV1 | pRC1 Vector |
| AAV6 | pRC6 Vector |

### (iii) pHelper Vector

### (iv) pAAV-GFP

After transfection, cultured in a CO₂ incubator at 37°C for 3 days.

### (2) Sampling of supernatant fraction AAV vector

After completion of the culture in Example 3-(1), a part of the culture medium was sampled and centrifuged (1,800×g, 10 min, 4°C). The supernatant after centrifugation was collected and used as "AAV vector supernatant fraction sample". After sampling, 0.5 M EDTA (pH 8.0) was added to the culture medium remaining in the well plate at 1/80 volume of the culture medium and mixed well. After reaction at room temperature for 10 min, the cells were detached. The detached cells were collected as the solution and centrifuged (1,800×g, 10 min, 4°C). After centrifugation, the supernatant was removed to give a cell pellet.

### (3) Sampling of cell fraction AAV vector

To the cell pellet of Example 3-(2) was added 80 µL of Extraction Solution A, and the cells were suspended by vortexing for 15 sec. After allowing to stand at room temperature for 5 min, the cells were further suspended by vortexing for 15 sec. The cell suspension was centrifuged (9,000×g, 10 min, 4°C). The cells were further suspended by vortexing for 15 sec. After allowing to stand at room temperature for 5 min, the cells were further suspended by vortexing for 15 sec. After centrifugation (9,000×g, 10 min, 4°C), the supernatant was collected, 8 µL of Extraction Solution B was added thereto and mixed well to give "AAV vector cell fraction sample".

### (4) Sampling of AAV vector with surfactant

As a sampling method of AAV vector different from Example 3-(2)(3), sampling using a surfactant was performed. Tween20 was added at a 1/1000 volume of the culture medium after completion of culture in Example 3-(1), 2 mM MgCl₂ was added to a final concentration and Endonuculease (KANEKA CORPORATION) was added at a final concentration of 50U/mL and they were stirred well. The cells were lysed by incubating in a CO₂ incubator at 37°C for 2 hr, and the culture medium was transferred into a centrifuge tube and centrifuged at 1,800×g for 10 min. The supernatant after centrifugation was used as an "AAV vector surfactant extraction sample (supernatant fraction+cell fraction)".

### Example 4

### Analysis of corresponding factor mRNA amount

The cultured cells of Example 2-(1) and Example 3-(1) were subjected to mRNA extraction using High pure RNA Isolation Kit (Roche) according to the manufacturer's protocol. A part of the RNA solution cleaned up by the column attached to the kit was taken, and the absorbance was measured using NanoDrop (Thermo Fisher Scientific, Inc.). Based on the measurement results, the RNA concentrations between the samples were adjusted, and cDNA was synthesized using the iScript AdV cDNA kit for RT-qPCR (BIO-RAD Laboratories, Inc.) according to the manufacturer's protocol.

Using the prepared cDNA solution as a template, real-time PCR was performed using THUNDERBIRD SYBR qPCR Mix (TOYOBO). For the real-time PCR reaction, CFX96Touch real-time PCR analysis system (Bio-Rad Laboratories, Inc.) was used. The amount of GAPDH mRNA was measured for each sample, and the measurement results of PIK3C2A, UBE2C, ASNS, NIFK, PDCD4, DDX47, GTPBP4, FTSJ3, DDX49, GNL2, NAT10, UBL5, SERBP1, MRE11, ISG15, CD44, LMNA, and ACP2 were normalized.

The ratio in each sample when the amount of corresponding factor mRNA in the control group (cells before regulation) is set to 1 is shown in Table 7, and the ratio of the GAPDH mRNA amount of PIK3C2A, UBE2C, ASNS, NIFK, PDCD4, DDX47, GTPBP4, DDX49, GNL2, NAT10, UBL5, SERBP1, ISG15, and LMNA in each sample when the amount of corresponding factor mRNA in the HEK293 cell (Accession number ATCC CRL1573) in the normal state (when AAV vector is not produced) is set to 1 is shown in Table 8.

**[Table 7]**

| factor name | Ratio |
|---|---|
| PIK3C2A | 38 |
| UBE2C | 37 |
| ASNS | 1.8.E+02 |
| NIFK | 32 |
| PDCD4 | 8.7.E+02 |
| DDX47 | 1.4.E+02 |
| GTPBP4 | 9.2.E+02 |
| FTSJ3 | 15 |
| DDX49 | 1.3.E+02 |
| GNL2 | 1.8.E+02 |
| NAT10 | 1.6 |
| UBL5 | 3.2 |
| SERBP1 | 49 |
| ISG15 | 0.58 |
| MRE11 | 0.73 |
| CD44 | 0.87 |
| LMNA | 0.39 |
| ACP2 | 0.64 |

**[Table 8]**

| factor name | Ratio |
|---|---|
| PIK3C2A | 5.9 |
| UBE2C | 15 |
| ASNS | 2.0.E+03 |
| NIFK | 43 |
| PDCD4 | 3.9.E+03 |
| DDX47 | 2.5.E+02 |
| GTPBP4 | 4.9.E+02 |
| DDX49 | 1.1.E+04 |
| GNL2 | 6.7.E+02 |
| NAT10 | 15.9 |
| UBL5 | 5.4 |
| SERBP1 | 19 |
| ISG15 | 0.09 |
| LMNA | 0.74 |

As shown in Table 7, in all the cells transfected with expression plasmid of PIK3C2A or UBE2C or ASNS or NIFK or PDCD4 or DDX47 or GTPBP4 or FTSJ3 or DDX49 or GNL2 or NAT10 or UBL5 or SERBP1 in Example 2-(1), the expression level of the corresponding factor was improved as compared with the control group (cells before regulation). In all the cells introduced with siRNA for MRE11 or ISG15 or CD44 or LMNA or ACP2 in Example 3-(1), the expression level of the corresponding factor was suppressed as compared with the control group (cells before regulation).

As shown in Table 8, the factor expression level was enhanced or suppressed as compared with HEK293 cells (Accession number ATCC CRL1573) in the normal state (when AAV vector is not produced).

### Example 5

### Evaluation of gene transfer efficiency of AAV vector

HEK293 cells (manufactured by Agilent, product number 240073) suspended in DMEM containing 10% FBS or HeLa cells (manufactured by ATCC, product number CCL-2) suspended in EMEM (WAKO) containing 10% FBS were seeded in a 24-well plate for cell culture (Corning Incorporated). Thereafter, the culture was continued in a CO₂ incubator at 37°C, and the cells were confirmed to be approximately 70-80% confluent.

The AAV vector cell fraction samples prepared in Example 2-(3) and Example 3-(3) for AAV2 vector, the AAV vector supernatant fraction sample prepared in Example 2-(2) for AAV1 vector, and the AAV vector surfactant extraction samples (supernatant fraction+cell fraction) prepared in Example 2-(4) and Example 3-(4) for AAV2 vector, AAV1 vector, and AAV6 vector were added to each well. Since the AAV2 vector is mainly present in the cell fraction and the AAV1 vector is mainly present in the supernatant fraction, the sample fraction to be used is changed according to the AAV vector serotype. After culturing in a COz incubator at 37°C for 3 days, the cells were subjected to flow cytometry analysis, the GFP positive rate in each sample was measured, and the ratio of gene transfer units (transduction units, hereinafter TU) to the control group was calculated. The results thereof are shown in Fig. 1, Fig. 2, Fig. 3, Fig. 4, Fig. 5, Fig. 6, Fig. 7, Fig. 8, Fig. 9, Fig. 10, and Fig. 11.

As shown in Fig. 1, Fig. 2, Fig. 3, Fig. 4, Fig. 5, and Fig. 6, more AAV vectors retaining infectivity as compared with the control group were produced due to the transient expression of PIK3C2A, UBE2C, ASNS, NIFK, PDCD4, DDX47, GTPBP4, FTSJ3, DDX49, GNL2, NAT10, UBL5, DNMT1, SERBP1, and NOVA2 at the time of AAV vector production. As shown in Fig. 7, Fig. 8, Fig. 9, Fig. 10, and Fig. 11, moreover, more AAV vectors retaining infectivity as compared with the control group were produced due to the knockdown of MRE11, ISG15, CD44, LMNA, and ACP2 at the time of AAV vector production.

From these results, it was found that the gene transfer efficiency of AAV vector can be improved by regulating the expression of a gene of any of ranks A to C listed in Table 1 and Table 2 in viral vector-producing cells.

### Example 6

### (1) AAV vector genome extraction

To the AAV vector supernatant fraction samples prepared in Example 2-(2), Example 3-(2), the AAV vector cell fraction samples prepared in Example 2-(3), Example 3-(3), and the AAV vector surfactant extraction samples (supernatant fraction+cell fraction) prepared in Example 2-(4), Example 3-(4) was added DNaseI (Takara Bio Inc.), and the mixture was reacted at 37°C for 15 min, and free genome and plasmid were removed. Then, a heat treatment was performed at 95°C for 10 min to deactivate DNaseI.

A 1/10 amount of Proteinase K (QIAGEN N.V.) and an equal amount of Buffer AL (QIAGEN N.V.) were added, and the mixture was allowed to stand at 56°C for 10 min, after which an equal amount of ethanol (Kanto Chemical Co., Inc.) was added and, using DNeasy Blood&Tissue Kit (QIAGEN N.V.), the AAV vector genome extract was cleaned up according to the manufacturer's protocol.

### (2) Genome titer measurement by real-time PCR

Using the AAV vector genome extract prepared in Example 6-(1) as a template, real-time PCR was performed using THUNDERBIRD SYBR qPCR Mix. As the standard for the calibration curve, solutions obtained by serially diluting linearized pAAV-GFP to 2×10⁷, 2×10⁶, 2×10⁵, 2×10⁴, 2×10³, 2×10² copies/µL were used. As the primer, one that amplifies the promoter region of the AAV vector genome was used. For the real-time PCR reaction, CFX96Touch real-time PCR analysis system (Bio-Rad Laboratories, Inc.) was used.

The AAV vector genome was quantified for the AAV vector supernatant fraction sample and the AAV vector cell fraction sample, the amount of AAV vector genome per unit culture bottom area in Example 2 and Example 3 (viral genome/cm², hereinafter vg/cm²) was calculated from the total value of the supernatant fraction and cell fraction, and the ratio to the control group was obtained. The AAV vector genome was quantified for the AAV vector surfactant extraction sample (supernatant fraction+cell fraction), the amount of AAV vector genome per unit culture bottom area (vg/cm²) in Example 2 and Example 3 was calculated, and the ratio to the control group (vg/ratio) was obtained. The results thereof are shown in Fig. 12, Fig. 13, Fig. 14, Fig. 15, Fig. 16, Fig. 17, Fig. 18, Fig. 19, Fig. 20, Fig. 21, and Fig. 22.

As shown in Fig. 12, Fig. 13, Fig. 14, Fig. 15, Fig. 16, and Fig. 17, higher AAV vector genome titers were obtained as compared with the control group due to the transient expression of PIK3C2A, UBE2C, ASNS, NIFK, PDCD4, DDX47, GTPBP4, FTSJ3, DDX49, GNL2, NAT10, UBL5, DNMT1, SERBP1, and NOVA2 at the time of AAV vector production. As shown in Fig. 18, Fig. 19, Fig. 20, Fig. 21, and Fig. 22, higher AAV vector genome titers per unit culture area was obtained as compared with the control group due to the knockdown of MRE11, ISG15, CD44, LMNA, and ACP2 at the time of AAV vector production.

From these results, it was found that the genome titer of AAV vector can be improved by regulating the expression of a gene of any of ranks A to C listed in Table 1 and Table 2 in viral vector-producing cells.

### Example 7

### (1) Transient expression of NIFK, SERBP1, and NOVA2 alone or in plurality in production cell

HEK293 cells (manufactured by Agilent, product number 240073) suspended in DMEM containing 10% FBS were seeded in a 6-well plate for cell culture (Corning Incorporated).

Thereafter, the culture was continued in a CO₂ incubator at 37°C, and the cells were confirmed to be approximately 70-80% confluent. The culture supernatant of the cells was removed, replaced with serum-free DMEM, and plasmids (i) to (iv) were transfected. Plasmids (ii) to (iv) and empty vector were transfected in the same manner into the cells of the control group.

### (i) Plasmid encoding corresponding factor

### (Vector BuilderInc., pRP-Neo-CAG-hNIFK Vector or pRP-Neo-CAG-hSERBP1 Vector, or pRP-Neo-CAG-hNOVA2 Vector)

**[Table 9]**

| corresponding factor name | plasmid name |
|---|---|
| NIFK | pRP-Neo-CAG-hNIFK Vector |
| SERBP1 | pRP-Neo-CAG-hSERBP1 Vector |
| NOVA2 | pRP-Neo-CAG-hNOVA2 Vector |
| NOVA2+NIFK | pRP-Neo-CAG-hNOVA2 Vector +pRP-Neo-CAG-hNIFK Vector |
| NOVA2+SERBP1 | pRP-Neo-CAG-hNOVA2 Vector +pRP-Neo-CAG-hSERBP1 Vector |

### (ii) pRC2-mi342 Vector

### (iii) pHelper Vector

### (iv) pAAV-GFP

After transfection, cultured in a CO₂ incubator at 37°C for 3 days.

### (2) Sampling of AAV vector with surfactant

Tween20 (Sigma Ltd.) was added at a 1/1000 volume of the culture medium after completion of culture in Example 7-(1), 2 mM MgCl₂ was added to a final concentration and Endonuculease (KANEKA CORPORATION) was added at a final concentration of 50U/mL and they were stirred well. The cells were lysed by incubating in a CO₂ incubator at 37°C for 2 hr, and the culture medium was transferred into a centrifuge tube and centrifuged at 1,800×g for 10 min. The supernatant after centrifugation was used as an "AAV vector surfactant extraction sample (supernatant fraction+cell fraction)".

### (3) Evaluation of gene transfer efficiency of AAV vector

HeLa cells suspended in EMEM containing 10% FBS were seeded in a 24-well plate for cell culture. Thereafter, the culture was continued in a CO₂ incubator at 37°C, and the cells were confirmed to be approximately 70-80% confluent.

The AAV vector surfactant extraction sample (supernatant fraction+cell fraction) prepared in Example 7-(1) was added to each well, cultured in a CO₂ incubator at 37°C for 3 days, and then subjected to flow cytometry analysis. The GFP positive rate in each sample was measured, and the ratio of gene transfer units (TU) to the control group was calculated. The results thereof are shown in Fig. 23 and Fig. 24.

As shown in Fig. 23, more AAV vectors retaining infectivity were produced due to the simultaneous transient expression of NOVA2 and NIFK at the time of AAV vector production, as compared with the control group and transient expression of NOVA2, NIFK alone. As shown in Fig. 24, moreover, more AAV vectors retaining infectivity were produced due to the simultaneous transient expression of NOVA2 and SERBP1 at the time of AAV vector production, as compared with the control group and transient expression of NOVA2, SERBP1 alone.

From these results, it was found that the gene transfer efficiency of AAV vector can be improved by regulating the expression of a gene of any of ranks A to C listed in Table 1 and Table 2 in viral vector-producing cells.

### Example 8

### (1) Selection of CD44 low expression cell

Considering that production cells with a higher viral vector production ability can be obtained using proteins with different expression levels of HEK293 cell and HEK293T cell in Table 1 and Table 2 as selection markers, selection of production cells using CD44 as a selection marker was performed as one embodiment.

A pellet of HEK293 cells (Accession number ATCC CRL1573) prepared at 1×10⁶ cells was suspended in 196 µL of 0.5% BSA (Sigma Ltd.) and 2 mM EDTA containing DPBS, 4 µL of APC-labeled anti CD44 antibody (Miltenyi Biotec) was added and mixed sufficiently, and the mixture was allowed to stand at 4°C for 10 min in the dark. 1 mL of DMEM containing 10% FBS and 1% MEM non-essential amino acid solution (100×) was added, and the mixture was centrifuged at 300×g, 4°C for 10 min, and the supernatant was removed. A cell pellet was suspended in DMEM containing 10% FBS and 1% MEM non-essential amino acid solution (100×), subjected to AS ONE Cell Picking System (AS ONE CORPORATION), and single cells with low APC luminance value were collected in a 384 plate. The recovered clone (clone A) was expanded in DMEM containing 10% FBS and 1% MEM non-essential amino acid solution (100×), and used in the subsequent tests.

### (2) CD44 expression level measurement of collected clones

In order to measure the expression level of CD44 on the cell surface of clone A obtained in Example 8-(1), a cell pellet of 5×10⁵ cells was prepared for clone A and HEK293 cells (Accession number ATCC CRL1573), which is a cell population before selection. The cell pellet was suspended in 98 µL of DPBS containing 0.5% BSA and 2 mM EDTA, 2 µL of APC-labeled anti CD44 antibody was added and mixed sufficiently, and the mixture was allowed to stand at 4°C for 10 min in the dark. 1 mL of DPBS containing 0.5% BSA and 2 mM EDTA was added, and the mixture was centrifuged at 300×g, 4°C for 10 min, and the supernatant was removed. The obtained cell pellet was suspended in 200 µL of DPBS containing 0.5% BSA and 2 mM EDTA and subjected to flow cytometric analysis. An APC fluorescence intensity histogram was obtained for each sample, and the results thereof are shown in Fig. 25.

As shown in Fig. 25, it was found that the expression level of CD44 on the cell surface of clone A was low as compared with HEK293 cell (Accession number ATCC CRL1573), which is a cell population before selection.

### (3) AAV vector production by collected clone

For evaluation of the AAV vector production ability of clone A obtained in Example 8-(1), AAV vector production test was performed as follows for clone A and HEK293 cells (Accession number ATCC CRL1573), which is a cell population before selection.

For evaluation of the AAV vector production ability of clone A suspended in DMEM containing 10% FBS and 1% MEM non-essential amino acid solution (100×), clone A and HEK293 cell (Accession number ATCC CRL1573), which is a cell population before selection, were seeded in a 6-well plate for cell culture. Thereafter, the culture was continued in a CO₂ incubator at 37°C, and the cells were confirmed to be approximately 70-80% confluent. The culture supernatant of the cells was removed, replaced with serum-free DMEM, and plasmids (i) to (iii) were transfected.

### (i) pRC2-mi342 Vector

### (ii) pHelper Vector

### (iii) pAAV-GFP

After transfection, cultured in a CO₂ incubator at 37°C for 3 days.

### (4) Sampling of AAV vector with surfactant

Tween20 was added at a 1/1000 volume of the culture medium after completion of culture in Example 8-(3), 2 mM MgClz was added to a final concentration and Endonuculease was added at a final concentration of 50U/mL and they were stirred well. The cells were lysed by incubating in a COz incubator at 37°C for 2 hr, and the culture medium was transferred into a centrifuge tube and centrifuged at 1,800×g for 10 min. The supernatant after centrifugation was used as an "AAV vector surfactant extraction sample (supernatant fraction+cell fraction)".

### (5) Evaluation of gene transfer efficiency of AAV vector

HeLa cells suspended in EMEM containing 10% FBS were seeded in a 24-well plate for cell culture. Thereafter, the culture was continued in a CO₂ incubator at 37°C, and the cells were confirmed to be approximately 70-80% confluent.

The AAV vector surfactant extraction sample (supernatant fraction+cell fraction) prepared in Example 8-(4) was added to each well, cultured in a CO₂ incubator at 37°C for 3 days, and then subjected to flow cytometry analysis. The GFP positive rate in each sample was measured, and the ratio of gene transfer units (TU) to HEK293 cells (Accession number ATCC CRL1573), a cell population before selection, was calculated. The results thereof are shown in Fig. 26.

As shown in Fig. 26, clone A, which is a CD44 low expression clone, produced more AAV vectors retaining infectivity as compared with HEK293 cell (Accession number ATCC CRL1573), which is a cell population before selection.

From these results, it was found that production cells with a higher AAV vector production ability than a cell population before selection can be obtained by using the proteins listed in Table 1 and Table 2 as selection markers in viral vector-producing cells.

### (6) AAV vector genome extraction

To the AAV vector surfactant extraction sample (supernatant fraction+cell fraction) prepared in Example 8-(4) was added DNaseI and the mixture was reacted at 37°C for 15 min, and free genome and plasmid were removed. Thereafter, 500 mM EDTA at a 1/10 volume of the DNaseI reaction mixture was added to quench the reaction of DNaseI.

Proteinase K at a 1/10 amount of the DNaseI reaction mixture and Buffer AL in an equal amount to the DNaseI reaction mixture were added, and the mixture was allowed to stand at 56°C for 10 min. Ethanol in an equal amount to the DNaseI reaction mixture was added and, using DNeasy Blood&Tissue Kit, the AAV vector genome extract was cleaned up according to the manufacturer's protocol

### (7) Genome titer measurement by digital PCR

Using the AAV vector genome extract prepared in Example 8-(6) as a template, digital droplet PCR was performed using ddPCR^{™} Supermix for Probes (No dUTP) (Bio-Rad Laboratories, Inc.). As the primer, one that amplifies the AAV vector genome ITR region and GFP region was used, FAM labeling probe was used for the ITR region, and HEX labeling probe was used for the GFP region. As the apparatus, QX200 AutoDG Droplet Digital PCR system (Bio-Rad Laboratories, Inc.) was used.

The AAV vector genome was quantified for the AAV vector surfactant extraction sample (supernatant fraction+cell fraction) from the number of droplets positive for both the ITR region and the GFP region, the amount of AAV vector genome per unit culture bottom area (vg/cm²) in Example 8-(3) was calculated, and the ratio to HEK293 cells (Accession number ATCC CRL1573), a cell population, is shown in Fig. 27.

As shown in Fig. 27, clone A, which is a CD44 low expression clone, showed a higher AAV vector genome titer as compared with HEK293 cell (Accession number ATCC CRL1573), which is a cell population before selection.

From these results, it was found that production cells with a higher AAV vector production ability than a cell population before selection can be obtained by using the proteins listed in Table 1 and Table 2 as selection markers in viral vector-producing cells.

### [Industrial Applicability]

The viral vector-producing cells obtained in the present invention show improve viral vector, particularly AAV vector, producing ability. Therefore, it becomes possible to safely improve of the producibility of viral vectors by using the viral vector-producing cell of the present invention as a packaging cell.

This application is based on a patent application No. 2020-217338 filed in Japan (filing date: December 25, 2020), the contents of which are incorporated in full herein by reference.

## Claims

1. A viral vector-producing cell with an improved ability to produce vectors, wherein expression of a protein derived from the viral vector-producing cell in the cell is regulated.

2. The viral vector-producing cell according to claim 1, wherein the expression of the protein derived from the viral vector-producing cell is regulated as compared with that in the viral vector-producing cell before regulation of the expression of the aforementioned protein.

3. The viral vector-producing cell according to claim 1, wherein the expression of the protein derived from the viral vector-producing cell is regulated as compared with that in HEK293 cell (Accession number ATCC CRL1573).

4. The viral vector-producing cell according to claim 1 or 2, wherein the aforementioned expression of the protein derived from the viral vector-producing cell is increased as compared with that in the viral vector-producing cell before regulation of the expression of the aforementioned protein.

5. The viral vector-producing cell according to claim 4, wherein the aforementioned expression of the protein derived from the viral vector-producing cell is increased 1.5 times or more at the time of viral vector production, as compared with that in the viral vector-producing cell before regulation of the expression of the aforementioned protein.

6. The viral vector-producing cell according to claim 1 or 3, wherein the aforementioned expression of the protein derived from the viral vector-producing cell is increased as compared with that in HEK293 cell (Accession number ATCC CRL1573).

7. The viral vector-producing cell according to claim 6, wherein the aforementioned expression of the protein derived from the viral vector-producing cell is increased 1.5 times or more at the time of viral vector production, as compared with that in the HEK293 cell (Accession number ATCC CRL1573).

8. The viral vector-producing cell according to any one of claims 4 to 7, wherein the increase in the aforementioned expression of the protein derived from the viral vector-producing cell is achieved by addition of an additive to the cell.

9. The viral vector-producing cell according to any one of claims 4 to 7, wherein the increase in the aforementioned expression of the protein derived from the viral vector-producing cell is achieved by introducing an exogenous plasmid into the viral vector-producing cell.

10. The viral vector-producing cell according to any one of claims 4 to 9, wherein the aforementioned protein derived from viral vector-producing cell is at least one type selected from the group consisting of PIK3C2A, UBE2C, ASNS, NIFK, PDCD4, DDX47, GTPBP4, FTSJ3, DDX49, GNL2, NAT10, UBL5, SERBP1, and NOVA2.

11. The viral vector-producing cell according to claim 1 or 2, wherein the aforementioned expression of the protein derived from the viral vector-producing cell is decreased as compared with that in the viral vector-producing cell before regulation of the expression of the aforementioned protein.

12. The viral vector-producing cell according to claim 11, wherein the aforementioned expression of the protein derived from the viral vector-producing cell is decreased by not less than 10% at the time of viral vector production, as compared with that in the viral vector-producing cell before regulation of the expression of the aforementioned protein.

13. The viral vector-producing cell according to claim 1 or 3, wherein the aforementioned expression of the protein derived from the viral vector-producing cell is decreased as compared with that in HEK293 cell (Accession number ATCC CRL1573).

14. The viral vector-producing cell according to claim 13, wherein the aforementioned expression of the protein derived from the viral vector-producing cell is decreased by not less than 10% at the time of viral vector production, as compared with that in the HEK293 cell (Accession number ATCC CRL1573).

15. The viral vector-producing cell according to any one of claims 11 to 14, wherein the decrease in the aforementioned expression of the protein derived from the viral vector-producing cell is achieved by addition of an additive to the cell.

16. The viral vector-producing cell according to any one of claims 11 to 14, wherein the decrease in the aforementioned expression of the protein derived from the viral vector-producing cell is achieved by introducing an exogenous plasmid into the viral vector-producing cell.

17. The viral vector-producing cell according to any one of claims 11 to 16, wherein the aforementioned protein derived from viral vector-producing cell is at least one type selected from the group consisting of ISG15, CD44, LMNA, ACP2 and MRE11.

18. The viral vector-producing cell according to any one of claims 1 to 17, which is derived from a human cell.

19. The viral vector-producing cell according to claim 18, wherein the aforementioned human cell is HEK293 cell.

20. The viral vector-producing cell according to any one of claims 1 to 19, wherein the aforementioned viral vector is an adeno-associated viral vector.

21. A method for producing a viral vector-producing cell with an improved ability to produce vectors, comprising regulating expression of a protein derived from the viral vector-producing cell in the viral vector-producing cell.

22. The production method according to claim 21, wherein the regulation is to regulate the expression of the aforementioned protein by comparison with the viral vector-producing cell before regulation.

23. The production method according to claim 21, wherein the regulation is to regulate the expression of the aforementioned protein by comparison with HEK293 cell (Accession number ATCC CRL1573).

24. The production method according to claim 21 or 22, wherein the regulation is to increase the aforementioned expression of the protein derived from the viral vector-producing cell as compared with that in the viral vector-producing cell before regulation of the expression of the aforementioned protein.

25. The production method according to claim 24, wherein the regulation is to increase the aforementioned expression of the protein derived from the viral vector-producing cell 1.5 times or more at the time of viral vector production, as compared with that in the viral vector-producing cell before regulation of the expression of the aforementioned protein.

26. The production method according to claim 21 or 23, wherein the regulation is to increase the aforementioned expression of the protein derived from the viral vector-producing cell, as compared with that in HEK293 cell (Accession number ATCC CRL1573).

27. The production method according to claim 26, wherein the regulation is to increase the aforementioned expression of the protein derived from the viral vector-producing cell 1.5 times or more at the time of viral vector production, as compared with that in the HEK293 cell (Accession number ATCC CRL1573).

28. The production method according to any one of claims 24 to 27, wherein the increase in the aforementioned expression of the protein derived from the viral vector-producing cell is achieved by addition of an additive to the cell.

29. The production method according to any one of claims 24 to 27, wherein the increase in the aforementioned expression of the protein derived from the viral vector-producing cell is achieved by introducing an exogenous plasmid into the viral vector-producing cell.

30. The production method according to any one of claims 24 to 29, wherein the aforementioned protein derived from the viral vector-producing cell is at least one type selected from the group consisting of PIK3C2A, UBE2C, ASNS, NIFK, PDCD4, DDX47, GTPBP4, FTSJ3, DDX49, GNL2, NAT10, UBL5, SERBP1, and NOVA2.

31. The production method according to claim 21 or 22, wherein the regulation is to decrease the aforementioned expression of the protein derived from the viral vector-producing cell as compared with that in the viral vector-producing cell before regulation of the expression of the aforementioned protein.

32. The production method according to claim 31, wherein the regulation is to decrease the aforementioned expression of the protein derived from the viral vector-producing cell by not less than 10% at the time of viral vector production, as compared with that in the viral vector-producing cell before regulation of the expression of the aforementioned protein.

33. The production method according to claim 21 or 23, wherein the aforementioned expression of the protein derived from the viral vector-producing cell is decreased as compared with that in the HEK293 cell (Accession number ATCC CRL1573).

34. The production method according to claim 33, wherein the regulation is to decrease the aforementioned expression of the protein derived from the viral vector-producing cell by not less than 10% at the time of viral vector production, as compared with that in the HEK293 cell (Accession number ATCC CRL1573).

35. The production according to any one of claims 21 to 34, wherein the decrease in the aforementioned expression of the protein derived from the viral vector-producing cell is achieved by addition of an additive to the cell.

36. The production method according to any one of claims 21 to 34, wherein the decrease in the aforementioned expression of the protein derived from the viral vector-producing cell is achieved by introducing an exogenous plasmid into the viral vector-producing cell.

37. The production method according to any one of claims 21 to 36, wherein the aforementioned protein derived from viral vector-producing cell is at least one type selected from the group consisting of ISG15, CD44, LMNA, ACP2 and MRE11.

38. The production method according to any one of claims 21 to 37, wherein the cell is derived from an animal cell.

39. The viral vector-producing cell according to claim 38, wherein the aforementioned human cell is HEK293 cell.

40. The production method according to any one of claims 21 to 39, wherein the aforementioned viral vector is an adeno-associated viral vector.

41. A method for enhancing a production amount of a viral vector in a viral vector-producing cell, comprising regulating expression of a protein derived from the viral vector-producing cell in the viral vector-producing cell.

42. The method according to claim 41, wherein the regulation is performed by comparison with the viral vector-producing cell before regulation of the expression of the aforementioned protein.

43. The method according to claim 41, wherein the regulation is performed by comparison with HEK293 cell (Accession number ATCC CRL1573) .

44. The method according to claim 41 or 42, wherein the regulation is to increase the aforementioned expression of the protein derived from the viral vector-producing cell as compared with that in the viral vector-producing cell before regulation of the expression of the aforementioned protein.

45. The method according to claim 41 or 43, wherein the regulation is to increase the aforementioned expression of the protein derived from the viral vector-producing cell, as compared with that in HEK293 cell (Accession number ATCC CRL1573) .

46. The method according to claim 44 or 45, wherein the increase in the aforementioned expression of the protein derived from the viral vector-producing cell is achieved by addition of an additive to the cell.

47. The method according to claim 44 or 45, wherein the increase in the aforementioned expression of the protein derived from the viral vector-producing cell is achieved by introducing an exogenous plasmid into the viral vector-producing cell.

48. The method according to claim 41 or 42, wherein the regulation is to decrease the aforementioned expression of the protein derived from the viral vector-producing cell as compared with that in the viral vector-producing cell before regulation of the expression of the aforementioned protein.

49. The method according to claim 41 or 43, wherein the aforementioned expression of the protein derived from the viral vector-producing cell is decreased as compared with that in the HEK293 cell (Accession number ATCC CRL1573) .

50. The method according to claim 48 or 49, wherein the decrease in the aforementioned expression of the protein derived from the viral vector-producing cell is achieved by addition of an additive to the cell.

51. The method according to claim 48 or 49, wherein the decrease in the aforementioned expression of the protein derived from the viral vector-producing cell is achieved by introducing an exogenous plasmid into the viral vector-producing cell.

52. A kit for producing a viral vector, comprising the viral vector-producing cells according to any one of claims 1 to 20 or viral vector-producing cells produced by the production method according to any one of claims 21 to 40.

53. A method for selecting a viral vector-producing cell with an improved ability to produce vectors, comprising a step of selecting a cell in which the expression of a protein derived from the viral vector-producing cell is regulated in the viral vector-producing cell.

54. The method according to claim 53, comprising a step of selecting a cell in which expression of the protein derived from the viral vector-producing cell is regulated in the viral vector-producing cells as compared with that in the viral vector-producing cell before regulation of the expression of the aforementioned protein.

55. The method according to claim 53, comprising a step of selecting a cell in which expression of the protein derived from the viral vector-producing cell is regulated in the viral vector-producing cells as compared with that in HEK293 cell (Accession number ATCC CRL1573).

56. The method according to claim 53 or 54, wherein the regulation is performed to increase the expression as compared with that in the viral vector-producing cell before regulation of the expression of the aforementioned protein.

57. The method according to claim 53 or 55, wherein the regulation is performed to increase the expression as compared with that in the HEK293 cell (Accession number ATCC CRL1573).

58. The method according to claim 53 or 54, wherein the regulation is performed to decrease the aforementioned expression of the protein derived from the viral vector-producing cell as compared with that in the viral vector-producing cell before regulation of the expression of the aforementioned protein.

59. The method according to claim 53 or 55, wherein the regulation is performed to decrease the aforementioned expression of the protein derived from the viral vector-producing cell, as compared with that in HEK293 cell (Accession number ATCC CRL1573).

60. The method according to claim 58 or 59, wherein the protein derived from the aforementioned viral vector-producing cells is CD44.
